(19) Europäisches Patentamt
European Patent Office
Office européen des brevets

(11) **EP 2 669 678 B1**

(12) **FASCICULE DE BREVET EUROPEEN**

(45) Date de publication et mention
de la délivrance du brevet:
**27.08.2014 Bulletin 2014/35**

(51) Int Cl.:
***G01N 33/49*** *(2006.01)*     ***G01N 15/14*** *(2006.01)*

(21) Numéro de dépôt: **13169989.4**

(22) Date de dépôt: **31.05.2013**

(54) **Procédé et système de caractérisation d'une variation de la vitesse de particules ou d'une agglomération de particules contenues dans un liquide, telles que des particules sanguines**

Verfahren und System zur Charakterisierung der Geschwindigkeitsschwankung oder Koagulation von Partikeln, die in einer Flüssigkeit enthalten sind, wie beispielsweise Blutpartikeln

Method and system for characterising the speed variation or clotting of particles in a liquid, such as blood particles

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **01.06.2012 FR 1255115**

(43) Date de publication de la demande:
**04.12.2013 Bulletin 2013/49**

(60) Demande divisionnaire:
**14177673.2**

(73) Titulaire: **Commissariat à l'Énergie Atomique et aux Énergies Alternatives**
**75015 Paris (FR)**

(72) Inventeurs:
• **Poher, Vincent**
**62340 Guines (FR)**
• **Cubizolles, Myriam-Laure**
**38700 Corenc (FR)**
• **Pouteau, Patrick**
**38240 Meylan (FR)**
• **Allier, Cédric**
**38000 Grenoble (FR)**
• **Spiaczka, Johanna**
**38650 Roissard (FR)**

(74) Mandataire: **Blot, Philippe Robert Emile**
**Cabinet Lavoix**
**2, place d'Estienne d'Orves**
**75441 Paris Cedex 09 (FR)**

(56) Documents cités:
**EP-A1- 2 233 923**    **US-A1- 2010 267 066**

• **DAN CHICEA: "Results of sediment motion visualization by a modified LASCA technique", PROCEEDINGS OF SPIE, S P I E - INTERNATIONAL SOCIETY FOR OPTICAL ENGINEERING, US, vol. 6785, 1 janvier 2007 (2007-01-01), pages 67851O-1, XP007918645, ISSN: 0277-786X, DOI: 10.1117/12.757883**
• **KALCHENKO V ET AL: "In vivo dynamic light scattering imaging of blood coagulation", JOURNAL OF BIOMEDICAL OPTICS, S P I E - INTERNATIONAL SOCIETY FOR OPTICAL ENGINEERING, US, vol. 12, no. 5, 12 septembre 2007 (2007-09-12), pages 52002-1, XP002551829, ISSN: 1083-3668, DOI: 10.1117/1.2778695 [extrait le 2007-09-12]**
• **SEEMANTINI K NADKARNI ET AL: "Characterization of Atherosclerotic Plaques by Laser Speckle Imaging", CIRCULATION, LIPPINCOTT WILLIAMS & WILKINS, US, vol. 112, 1 janvier 2005 (2005-01-01), pages 885-892, XP007918646, ISSN: 0009-7322, DOI: 10.1161/CIRCULATIONAHA.104.520098 [extrait le 2005-08-01]**
• **PIEDERRIÈRE Y ET AL: "Evaluation of blood plasma coagulation dynamics by speckle analysis", JOURNAL OF BIOMEDICAL OPTICS, S P I E - INTERNATIONAL SOCIETY FOR OPTICAL ENGINEERING, US, vol. 9, no. 2, 1 mars 2004 (2004-03-01), pages 408-412, XP002551759, ISSN: 1083-3668, DOI: 10.1117/1.1645799 [extrait le 2004-03-22]**

**Description**

La présente invention concerne un procédé de caractérisation d'une variation de la vitesse de particules ou d'une agglomération de particules, les particules, telles que des particules sanguines, étant contenues dans un liquide, le procédé comportant les étapes suivantes :

- l'introduction du liquide dans une chambre fluidique ;
- l'éclairage de la chambre fluidique via un faisceau lumineux d'excitation émis par une source de lumière, le faisceau lumineux s'étendant à travers la chambre fluidique selon une direction longitudinale ;
- l'acquisition d'au moins deux images par un photodétecteur matriciel, les images étant formées par un rayonnement transmis par la chambre fluidique éclairée ; et
- le calcul, à partir des images acquises d'au moins un indicateur caractérisant la variation de la vitesse ou l'agglomération des particules.

L'invention concerne également un système de caractérisation de la variation de la vitesse des particules ou de l'agglomération des particules contenues dans le liquide, telles que des particules sanguines.

L'invention concerne notamment le domaine de l'imagerie sans lentille de focalisation du faisceau lumineux éclairant la chambre fluidique, afin de caractériser un liquide, tel que le sang.

L'invention s'applique en particulier à la détermination d'un paramètre concernant la coagulation du sang, notamment la mesure du temps de coagulation. Elle s'applique également à la détermination d'un paramètre concernant l'agglutination de particules dans le sang, notamment la détermination du groupe sanguin en caractérisant une agrégation cellulaire entre le sang à tester et un anticorps.

Kalchenko, V. et al, Journal of Biomedical Optics, 12(5), 052002 (2007) and Piederrière, Y et al, Journal of Biomedical Optics, 9(2), 408-412 (2004) décrivent l'analyse de sang par des techniques optiques.

On connaît du document EP 2 233 923 A1 un procédé et un système de caractérisation du type précité. Le procédé décrit vise à caractériser la dynamique de coagulation ou de sédimentation d'un fluide contenant du sang. Le système pour la mise en oeuvre de ce procédé comprend une chambre fluidique de réception du liquide, une source de lumière spatialement cohérente propre à émettre un faisceau lumineux d'éclairement et un miroir de réflexion du faisceau lumineux en direction de la chambre. Le faisceau lumineux s'étend selon une direction longitudinale depuis le miroir de réflexion vers la chambre fluidique.

Le système comprend également un capteur d'images, tel qu'un capteur matriciel de type CCD (de l'anglais *Charged-Coupled Device*) ou CMOS (de l'anglais *Complementary Metal Oxyde Semi-conductor*), agencé pour permettre l'acquisition d'une série temporelle d'images d'un motif optique de granularité engendré par l'interaction entre les particules contenues dans la chambre et le faisceau lumineux. Le système de caractérisation comprend également une unité de traitement de ladite série temporelle d'images.

La chambre fluidique est disposée entre le miroir et le capteur d'images selon la direction longitudinale. La distance entre la chambre fluidique et le capteur d'images selon la direction longitudinale est de quelques centimètres ou dizaines de centimètres. Le faisceau lumineux émis par la source de lumière spatialement cohérente présente une surface comprise entre 10 $\mu m^2$ et quelques $mm^2$ suivant un plan perpendiculaire à la direction longitudinale et traversant la chambre fluidique.

Un tel système et un tel procédé permettent de caractériser efficacement la dynamique de coagulation ou de sédimentation du sang contenu dans le liquide.

Toutefois, un tel système est assez encombrant. De plus, il permet d'observer le phénomène de coagulation seulement dans un volume relativement réduit de la chambre fluidique.

Le but de l'invention est donc de proposer un procédé et un système de caractérisation permettant d'observer un plus grand volume de liquide tout en limitant l'encombrement du système de caractérisation.

A cet effet, l'invention a pour objet un procédé de caractérisation selon la revendication 1.

Suivant d'autres aspects avantageux de l'invention, le procédé de caractérisation comprend une ou plusieurs des caractéristiques selon les revendications dépendantes 2 à 16 attenantes.

L'invention a également pour objet un système de caractérisation de la variation de la vitesse de particules ou de l'agglomération de particules, selon la revendication 17.

Suivant un autre aspect avantageux de l'invention, le photodétecteur matriciel comporte la caractéristique selon la revendication dépendante 18 attenante.

Ces caractéristiques et avantages de l'invention apparaîtront à la lecture de la description qui va suivre, donnée uniquement à titre d'exemple non limitatif, et faite en référence aux dessins annexés, sur lesquels :

- la figure 1 est une représentation très schématique d'un système de caractérisation selon l'invention, comprenant une chambre fluidique de réception du liquide à caractériser, une source de lumière propre à éclairer la chambre suivant une direction longitudinale, un photodétecteur matriciel d'acquisition d'images du rayonnement transmis par la chambre éclairée et une unité de traitement d'informations,

- la figure 2 est une représentation très schématique du système de caractérisation selon l'invention, suivant un autre agencement de la source de lumière par rapport au photodétecteur matriciel,
- la figure 3 est une vue schématique de la chambre fluidique selon la direction longitudinale, ainsi que de l'agencement du photodétecteur matriciel de la figure 1 par rapport à la chambre, selon une première variante,
- la figure 4 est une vue analogue à celle de la figure 3 selon une deuxième variante,
- la figure 5 est un organigramme d'un procédé de caractérisation selon l'invention,
- la figure 6 est une image d'un canal vide de la chambre de la figure 1, acquise par le photodétecteur matriciel,
- les figures 7 et 8 sont des images de la chambre contenant le liquide, acquises par le photodétecteur à différents instants temporels,
- les figures 9 et 10 sont des images de corrélation, calculées par l'unité de traitement de la figure 1, à partir des images acquises à différents instants temporels,
- la figure 11 est une représentation de l'évolution temporelle d'un indicateur caractérisant une variation de la vitesse des particules contenues dans le liquide, telle qu'un ralentissement des particules,
- la figure 12 est un tableau illustrant les cas d'agrégations cellulaires en fonction du groupe sanguin et de l'anticorps déposés,
- la figure 13 est une vue analogue celle des figures 3 et 4 selon un deuxième mode de réalisation, la chambre fluidique comportant deux canaux,
- les figures 14 et 15 sont des images respectives des premier et deuxième canaux de la chambre de la figure 13, le deuxième canal présentant une agrégation cellulaire, les images étant acquises par le photodétecteur de la figure 1,
- les figures 16 et 17 sont des histogrammes du niveau de gris des images acquises des figures 14 et 15,
- les figures 18 à 21 sont des images du liquide à caractériser, acquises par le photodétecteur selon un deuxième exemple du deuxième mode de réalisation, le liquide à caractériser contenant du sang, auquel une quantité variable d'anticorps est ajoutée, ces images étant acquises pour des quantités croissantes d'anticorps,
- les figures 22 à 25 sont des histogrammes du niveau de gris des images acquises des figures 18 à 21 respectivement,
- les figures 26 à 29 sont des images du liquide caractérisé sur les figures 18 à 21 respectivement, obtenues, après dilution, à l'aide d'un microscope et formant des images de référence,
- les figures 30 à 34 sont des images du liquide à caractériser, acquises par le photodétecteur selon un troisième exemple du deuxième mode de réalisation, le liquide à caractériser contenant du sang, une quantité variable de protéine A, et une même quantité d'anticorps ajoutée,
- les figures 35 à 39 sont des histogrammes du niveau de gris des images acquises des figures 30 à 34 respectivement,
- les figures 40 à 44 sont des images du liquide caractérisé sur les figures 30 à 34 respectivement, obtenues après dilution à l'aide d'un microscope et formant des images de référence, et
- la figure 45 est une représentation très schématique de l'agglutination des globules rouges et des protéines A à l'aide des anticorps.

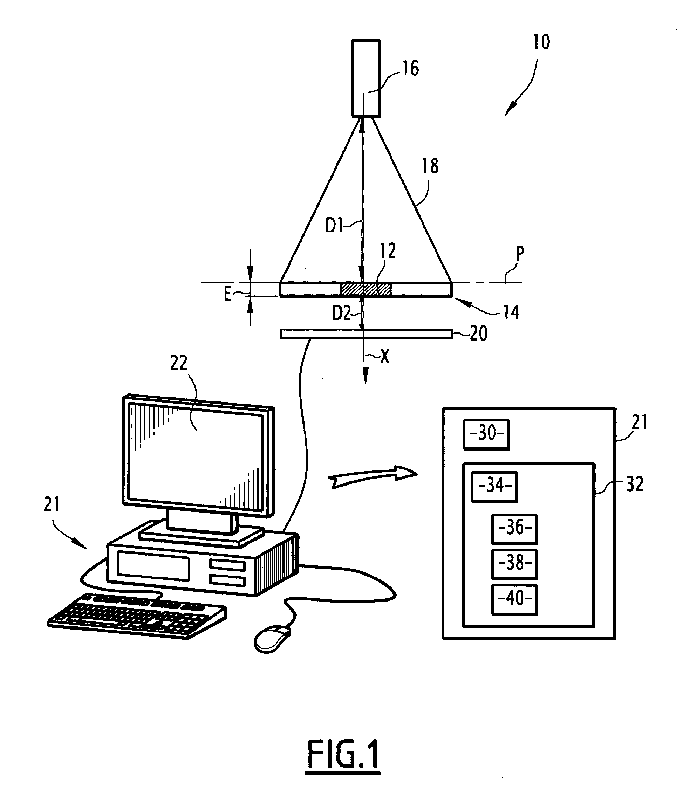

Sur la figure 1, un système de caractérisation 10 est destiné à caractériser une variation de la vitesse de particules ou l'agglomération de particules, les particules, telles que des particules sanguines, étant contenues dans un liquide 12, via l'acquisition d'images formées par un rayonnement transmis par le liquide 12 éclairé, puis le traitement de ces images. La variation de la vitesse de particules est, par exemple, un ralentissement des particules tel que cela sera décrit plus en détail par la suite. L'homme du métier comprendra que le système de caractérisation 10 selon l'invention est de manière analogue propre à caractériser une accélération des particules.

Ainsi, de façon générale, le système de caractérisation 10 est destiné à caractériser un paramètre d'un liquide comprenant des particules, ce liquide étant notamment du sang. Ce paramètre est, par exemple, une coagulation ou encore une agglomération de particules constituant le liquide. En variante, il s'agit d'un comptage de particules ou d'une observation de la morphologie de particules.

Par particule, on entend, par exemple, une particule biologique, c'est-à-dire une cellule (par exemple un globule rouge, un globule blanc, ou une plaquette), une bactérie, un virus ou encore toute autre molécule (par exemple une protéine).

Par agglutination (ou agglomération), on entend la formation d'un édifice tridimensionnel de particules reliées entre elles, sous l'effet d'un réactif introduit.

Par état d'agglutination (ou d'agglomération), on entend une estimation, relative ou absolue, de la taille des agglutinats ou relative à la quantité de particules présentes dans les agglutinats.

Le système de caractérisation 10 comprend une chambre fluidique 14 destinée à recevoir le liquide 12, une source de lumière 16 propre à émettre un faisceau lumineux d'excitation 18 pour éclairer la chambre fluidique 14, le faisceau lumineux 18 dirigé selon une direction longitudinale X à travers la chambre fluidique 14, et un photodétecteur matriciel 20 propre à acquérir des images du rayonnement transmis par la chambre fluidique 14 éclairée par le faisceau lumineux 18. Par rayonnement transmis, on entend le rayonnement traversant la chambre fluidique, de telle sorte que le photodétecteur matriciel 20 et la source de lumière 16 sont situés de part et d'autre de la chambre fluidique 14.

Le système de caractérisation 10 comprend une unité de traitement d'informations 21 et un écran 22 d'affichage d'une image de la chambre 14.

Dans le mode de réalisation décrit, le système de caractérisation 10 est propre à caractériser la coagulation du sang ou l'agglutination de particules sanguines, l'agglutination de particules sanguines permettant de déterminer le groupe sanguin associé. Le liquide 12 contient alors du sang. Le liquide 12 est, par exemple, du sang entier, une fraction du sang, ou encore un plasma sanguin. En variante, le liquide 12 est un autre liquide corporel, tel que l'urine, la sueur...

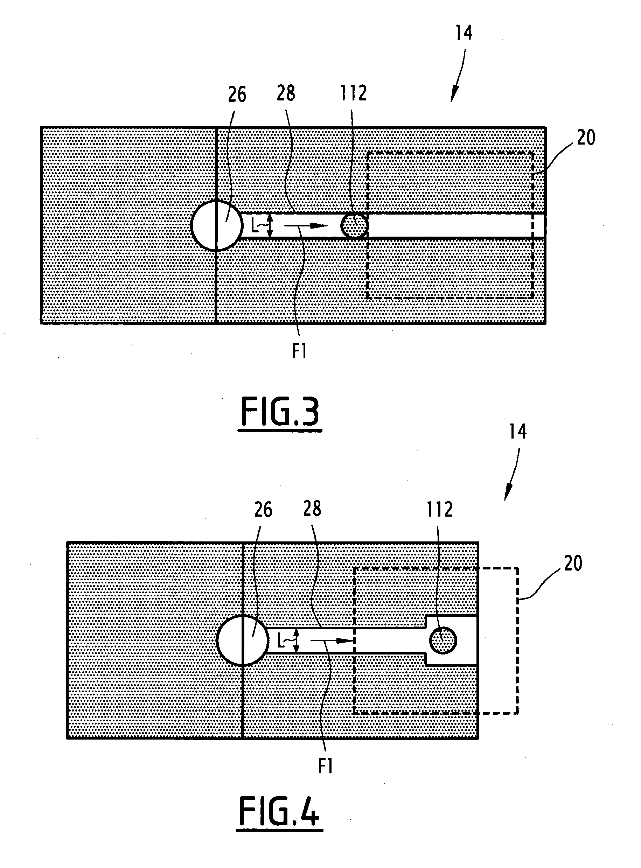

La chambre fluidique 14 est disposée entre la source de lumière 16 et le photodétecteur matriciel 20 selon la direction longitudinale X. La chambre fluidique 14 comprend une zone 26 de dépôt du liquide et un ou plusieurs canaux 28 de circulation du liquide 12, comme représenté sur la figure 3.

La chambre fluidique 14 comporte au moins un canal fluidique, délimité, selon la direction X, par une plaque supérieure et une plaque inférieure, non représentées. Ces plaques sont au moins partiellement translucides afin de permettre l'éclairement du liquide 12 par la source de lumière 16, ainsi que la détection du rayonnement transmis par le détecteur matriciel 20.

Les plaques inférieure et supérieure sont, par exemple, deux lames de verre, non représentées et séparées par des espaceurs non représentés, de sorte que les lames de verre sont espacées d'environ 160 $\mu$m selon la direction longitudinale X.

La chambre fluidique 14 présente une épaisseur E selon la direction longitudinale X. L'épaisseur E est, par exemple, de valeur comprise entre 20 $\mu$m et 1000 $\mu$m, de préférence comprise entre 30 $\mu$m et 300 $\mu$m.

La source de lumière 16 est propre à émettre le faisceau lumineux 18 selon la direction longitudinale X.

La source de lumière 16 est disposée à une première distance D1 de la chambre fluidique 14 selon la direction longitudinale X. La première distance D1 présente, de préférence, une valeur comprise entre 1 cm et 30 cm, par exemple égale à 20 cm.

Dans le mode de réalisation décrit, la source de lumière 16 est une source spatialement et temporellement cohérente. La source de lumière 16 est, par exemple, un laser. En variante, la source de lumière 16 est une diode Laser (DL) ou encore une diode Laser de type VCSEL (de l'anglais *Vertical Cavity Surface Emitting Laser*).

En variante encore, la source de lumière 16 est une diode électroluminescente, également appelée LED (de l'anglais *Light-Emitting Diode*), monochromatique et présentant des dimensions suffisamment réduites pour être considérée comme spatialement cohérente, le diamètre de la LED étant inférieur au dixième de la première distance D1 séparant cette LED de la chambre.

Le faisceau lumineux 18, orienté selon la direction longitudinale X, présente au niveau de la chambre fluidique, une surface comprise entre 5 mm$^2$ et 200 mm$^2$, de préférence égale à 25 mm$^2$, suivant un plan P perpendiculaire à la direction longitudinale X, comme représenté sur la figure 1. Le plan P est agencé au contact de la chambre fluidique 14. Ainsi, la surface de fluide éclairée est plus importante que dans l'état de la technique. Cela permet de s'affranchir de fluctuations locales du paramètre que l'on souhaite déterminer.

Le faisceau lumineux 18 est propre à éclairer directement la chambre fluidique 14, de préférence en l'absence d'une optique de grossissement disposée entre la source de lumière 16 et la chambre fluidique 14.

Le photodétecteur matriciel 20 est un capteur d'images pixélisé, comportant une pluralité de pixels, non représentés. Chaque pixel du photodétecteur 20 présente des dimensions inférieures ou égales à 10 $\mu$m, voire 4 $\mu$m. Chaque pixel est, par exemple, en forme d'un carré dont le côté est de valeur inférieure ou égale à 10 $\mu$m, voire 4 $\mu$m. Dans le mode de réalisation décrit, chaque pixel est en forme d'un carré de 4 $\mu$m de côté. En variante, chaque pixel est en forme d'un carré de 2,2 $\mu$m de côté.

Le photodétecteur matriciel 20 est disposé à une deuxième distance D2 de la chambre fluidique 14 selon la direction longitudinale X. La deuxième distance D2 présente une valeur inférieure à 1 cm, et de préférence comprise entre 100 $\mu$m et 2 mm. Le fait de privilégier une distance courte entre le détecteur et la chambre permet de limiter les phénomènes d'interférence entre les différentes figures de diffraction. En effet, lorsque cette distance augmente, ces interférences sont susceptibles de rendre l'image inexploitable, notamment lorsque le nombre de particules diffractantes augmente. Cela est dû au fait que, le volume de fluide éclairé est plus important que dans le dispositif décrit dans la demande EP 2 233 923 A1 de l'état de la technique. En plaçant le détecteur à une distance supérieure à 1cm, l'image obtenue sur le détecteur serait difficilement exploitable

Les images acquises par le photodétecteur matriciel 20 sont formées par le rayonnement transmis directement par la chambre fluidique 14 éclairée, en l'absence d'une optique de grossissement disposée entre la chambre fluidique 14 et le photodétecteur matriciel 20. Le photodétecteur matriciel 20 est également appelé dispositif d'imagerie sans lentille, et est apte à former une image de la chambre fluidique 14, tout en étant placé à une faible distance de cette dernière. Par faible distance, on entend une distance inférieure à 1 cm.

Le photodétecteur matriciel 20 est propre à générer au moins une image toutes les 5 secondes, et la cadence d'acquisition est donc supérieure à 0,2 Hz. Le photodétecteur matriciel 20 est un capteur d'images en deux dimensions, à savoir dans un plan perpendiculaire à l'axe longitudinal X. La fréquence d'acquisition des images est de préférence comprise entre 1 Hz et 20 Hz.

Le photodétecteur matriciel 20 est, par exemple, un capteur CCD. En variante, le photodétecteur 20 est un capteur CMOS.

Le photodétecteur matriciel 20 est, par exemple, sensiblement aligné avec la chambre fluidique 14 selon la direction longitudinale X, comme illustré sur la figure 3 où le photodétecteur 20 est représenté en traits pointillés.

En variante, le photodétecteur matriciel 20 est légèrement décalé par rapport à la chambre 14 suivant l'axe longitudinal X, comme illustré sur la figure 4 où le photodétecteur 20 est également représenté en traits pointillés.

L'unité de traitement d'informations 21, visible sur la figure 1, comporte un processeur de données 30 et une mémoire 32 associée au processeur.

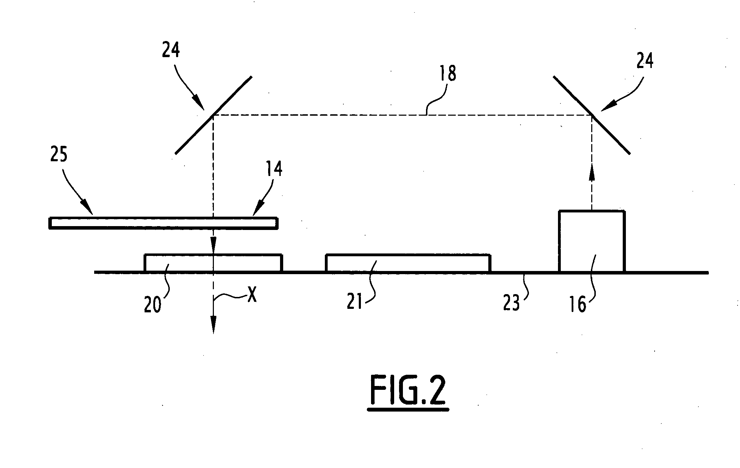

Dans l'exemple de réalisation de la figure 2, le photodétecteur matriciel 20, la source de lumière 16 et éventuellement tout ou partie de l'unité de traitement d'informations 21 sont solidaires d'un même substrat 23. Le système de caractérisation 10 comprend un système optique 24, par exemple un miroir, permettant de renvoyer le faisceau lumineux 18 de la source de lumière 16 vers le photodétecteur 20. Cela permet de disposer d'un système compact. La chambre fluidique 14 est, par exemple, ménagée dans un support amovible 25. Le support amovible 25 est, par exemple, jetable et est destiné à être inséré à l'aplomb du photodétecteur 20, à faible distance de ce dernier, de telle sorte que la chambre fluidique est apte à être éclairée par le faisceau lumineux 18. Selon cet exemple de réalisation, le support 25 est destiné à recevoir le fluide à analyser 12, puis à être inséré à proximité du photodétecteur 20 pour que l'analyse puisse être réalisée. Il comprend par exemple un conduit, dans lequel le fluide 12 circule jusqu'au canal 28 de la chambre fluidique, la chambre fluidique 14 étant raccordée à ce conduit. Lorsque l'analyse est terminée, le support 25 est retiré pour être, notamment, jeté. Le système de caractérisation 10 est alors disponible pour réaliser une autre mesure avec un autre support.

L'homme du métier comprendra que, dans l'exemple de réalisation de la figure 2, la direction longitudinale X correspond à la dernière portion du faisceau lumineux 18 entre le miroir correspondant du système optique 24 et le photodétecteur 20, traversant la chambre fluidique 14.

Le ou chaque canal de circulation 28 présente une largeur L, visible sur les figures 3 et 4. La largeur L est, par exemple, de valeur comprise entre 50 $\mu$m et 5 mm, de préférence égale à 1,5 mm.

La mémoire 32 est apte à stocker un logiciel 34 de réception des images acquises par le photodétecteur matriciel 20, un premier logiciel 36 de calcul d'un premier indicateur $Ind1_{n,n+m}$ apte à caractériser le paramètre recherché; en l'occurrence la variation de la vitesse des particules, telle que leur ralentissement. En complément ou en variante, la mémoire 32 est apte à stocker un deuxième logiciel 38 de calcul d'un deuxième indicateur $Ind2$ apte à caractériser un autre paramètre recherché, en l'occurrence l'agglomération des particules. La mémoire 32 est également apte à stocker un logiciel 40 de caractérisation de la variation de la vitesse des particules et/ou de l'agglomération des particules.

En variante, les moyens de réception 34, les premiers moyens de calcul 36, les deuxièmes moyens de calcul 38 et les moyens de caractérisation 40 sont réalisés sous forme de composants logiques programmables ou encore sous forme de circuits intégrés dédiés.

Le logiciel de réception 34 est propre à recevoir régulièrement de la part du photodétecteur 20 les images acquises séquentiellement, à différents instants. Le logiciel de réception 34 est propre à recevoir au moins une image par seconde, et la cadence de réception des images est supérieure à 0,2 Hz, typiquement dans la plage de 1 Hz à 20 Hz.

Le premier logiciel de calcul 36 est propre à calculer une image $A_n$, représentant l'image de transmission $I_n(x,y)$, à laquelle est retranchée une moyenne locale. Cette dernière est obtenue en convoluant l'image $I_n(x,y)$ avec un noyau k1. Ce noyau k1 est une matrice de dimensions faibles par rapport à $I_n$. Par exemple, les dimensions du noyau k1 sont de 10 pixels par 10 pixels, et les dimensions de $I_n$ sont au moins deux fois supérieures à celles du noyau k1, voire 10 fois supérieures. Le noyau k1, comportant P lignes et Q colonnes, est, par exemple, homogène, toutes ses valeurs étant identiques. D'après ce qui précède, P et Q sont des nombres entiers, par exemple égaux à 10. Ainsi, on établit deux images $A_n$ et $A_{n+m}$, correspondant respectivement aux instants n et n+m, m étant un entier. En général, m est égal à 1, les images de transmission $I_n$ et $I_{n+1}$ étant deux images de transmission successives.

$$A_n(x, y) = I_n(x, y) - (I_n \otimes k1)(x, y) \qquad\qquad (1)$$

$$A_{n+m}(x, y) = I_{n+m}(x, y) - (I_{n+m} \otimes k1)(x, y) \qquad\qquad (2)$$

où $I_n(x, y)$, $I_{n+m}(x, y)$ représentent deux images de transmission successives aux instants n et n+m, x et y représentant les coordonnées d'un point de l'image respective, $I_n(x,y)$, $I_{n+m}(x,y)$ étant des matrices ayant X lignes et Y colonnes, le symbole $\otimes$ représentant le produit de convolution défini par l'équation suivante :

$$(F \otimes k1)(x, y) = \sum_{p=0}^{P} \sum_{q=0}^{Q} F(x - p, y - q) k1(p, q) \qquad (3)$$

F étant une matrice à X lignes et Y colonnes,

k1 représentant un noyau pour la corrélation des images acquises, k1 étant une matrice à P lignes et Q colonnes,

X, Y, P et Q étant des nombres entiers vérifiant $X \geq P \geq 1$ et $Y \geq Q \geq 1$.

Les images sont, par exemple, acquises toutes les secondes par le photodétecteur matriciel 20, et les deux images de transmission $I_n(x,y)$, $I_{n+1}(x,y)$ sont alors des images acquises à une seconde d'intervalle.

Le premier logiciel de calcul 36 est ensuite propre à calculer une image de corrélation $Icorr_{n,n+m}(x,y)$ représentative de la corrélation entre deux images de transmission $I_n(x,y)$, $I_{n+1}(x,y)$ par exemple selon l'équation suivante :

$$Icorr_{n,n+m}(x, y) = \frac{\left((A_n \times A_{n+m}) \otimes k1\right)(x, y)}{\sqrt{\left({A_n}^2 \otimes k1\right)(x, y)} \sqrt{\left({A_{n+m}}^2 \otimes k1\right)(x, y)}} \qquad (4)$$

où $Icorr_{n,n+m}(x,y)$ représente l'image de corrélation de deux images de transmission $I_n$, $I_{n+m}$ établies aux instants respectifs n et n+m ; x et y représentent les coordonnées d'un point de l'image, $Icorr_{n,n+m}(x,y)$ étant une matrice ayant X lignes et Y colonnes.

Le premier logiciel de calcul 36 est enfin propre à calculer le premier indicateur $Ind1_{n,n+m}$ à partir de l'image de corrélation $Icorr_{n,n+m}(x,y)$ obtenue précédemment. Cet indicateur $Ind1_{n,n+m}$ est représentatif de l'intensité de l'image $Icorr_{n,n+m}(x,y)$. Cet indicateur $Ind1_{n,n+m}$ est alors apte à caractériser la variation de la vitesse des particules, telle que leur ralentissement.

L'indicateur de corrélation $Ind1_{n,n+m}$ est représentatif de la corrélation entre au moins deux images de transmission $I_n(x,y)$ et $I_{n+m}(x,y)$ respectivement acquises aux instants n et n+m, cette corrélation étant établie pour une région d'intérêt 142 de l'image de corrélation $Icorr_{n,n+m}(x,y)$. Ladite région d'intérêt 142 est déterminée par l'utilisateur. Elle correspond à la zone de l'image de corrélation $Icorr_{n,n+m}(x,y)$ que l'on utilise pour déterminer l'indicateur de corrélation $Ind1_{n,n+m}$. Il s'agit par exemple d'une zone carrée comportant quelques dizaines de pixels de côté, par exemple 50 x 50 pixels. L'indicateur de corrélation $Ind1_{n,n+m}$ traduit la valeur l'intensité dans cette région d'intérêt 142. Il est notamment déterminé à partir de l'intensité moyenne ou l'intensité totale dans la région d'intérêt 142 de l'image $Icorr_{n,n+m}(x,y)$. Cet indicateur $Ind1_{n,n+m}$ représente, par exemple, le niveau de l'intensité moyenne ou de ladite intensité totale dans la région d'intérêt 142.

En variante, le premier logiciel de calcul 36 est propre à calculer des images intermédiaires $C_n(x,y)$, $C_{n+m}(x,y)$ à partir des deux images de transmission $I_n(x,y)$, $I_{n+m}(x,y)$ acquises à des instants n et n+m, selon l'équation suivante :

$$C_n(x, y) = I'_n(x, y) - \overline{I'_n} \qquad (5)$$

$$C_{n+m}(x, y) = I'_{n+m}(x, y) - \overline{I'_{n+m}} \qquad (6)$$

où $I'_n(x,y)$, $I'_{n+m}(x,y)$ représentent respectivement une région d'intérêt des deux images de transmission $I_n$ et $I_{n+m}$. Comme précédemment évoqué, l'indice m est, par exemple, égal à 1. Les coordonnées x et y désignent les coordonnées d'un point de l'image, $C_n(x,y)$, $C_{n+m}(x,y)$ étant des matrices ayant N lignes et M colonnes, et

$\overline{I'}$, $\overline{I'}$ représentent une valeur moyenne des régions d'intérêt respectives $I_{n'}(x,y)$, $I_{m'}(x,y)$. Précisons que la soustraction de $I'_n$ et de $I'_m$ respectivement par $\overline{I'}$ et $\overline{I'}$ est facultative Cela correspond à une étape de normalisation, permettant d'obtenir un indicateur compris entre 0 et 1. De plus, cela permet de s'affranchir de l'effet de fluctuation de l'intensité d'éclairement du milieu entre deux images.

Selon cette variante, le premier logiciel de calcul 36 est alors propre à calculer le premier indicateur $Ind1_{n,n+m}$ selon l'équation suivante :

$$Ind1_{n,n+m} = \frac{\sum_{x=1,y=1}^{N,M} \sqrt{C_n^2(x,y)}\sqrt{C_{n+m}^2(x,y)}}{\sqrt{\sum_{x=1,y=1}^{N,M} C_n^2(x,y)}\sqrt{\sum_{x=1,y=1}^{N,M} C_{n+m}^2(x,y)}} \qquad (7)$$

où $Ind1_{n,n+m}$ représente le premier indicateur.

Le logiciel de caractérisation 40 est propre à caractériser la variation de la vitesse et/ou l'agglomération de particules contenues dans le liquide 12. Plus précisément, le logiciel de caractérisation 40 est propre à déterminer, à partir du premier indicateur calculé $Ind1_{n,n+m}$, la variation de la vitesse des particules contenues dans le liquide 12, telle que leur ralentissement. Dans le mode de réalisation décrit où le liquide 12 contient du sang, le premier logiciel de caractérisation 40 est propre à déterminer, à partir du premier indicateur calculé $Ind1_{n,n+m}$ la coagulation des particules sanguines et/ou un intervalle temporel, dit temps de coagulation, entre un instant d'origine et l'instant auquel le premier indicateur calculé $Ind1_{n,n+m}$ prend une valeur prédéterminée. Ainsi, d'une façon générale, $Ind1_{n,n+m}$ caractérise un paramètre de coagulation du sang, basé sur l'observation d'images de transmission $I_n$, $I_{n+m}$ aux instants n et n+m, m étant généralement compris entre 1 et 10, et de préférence égal à 1.

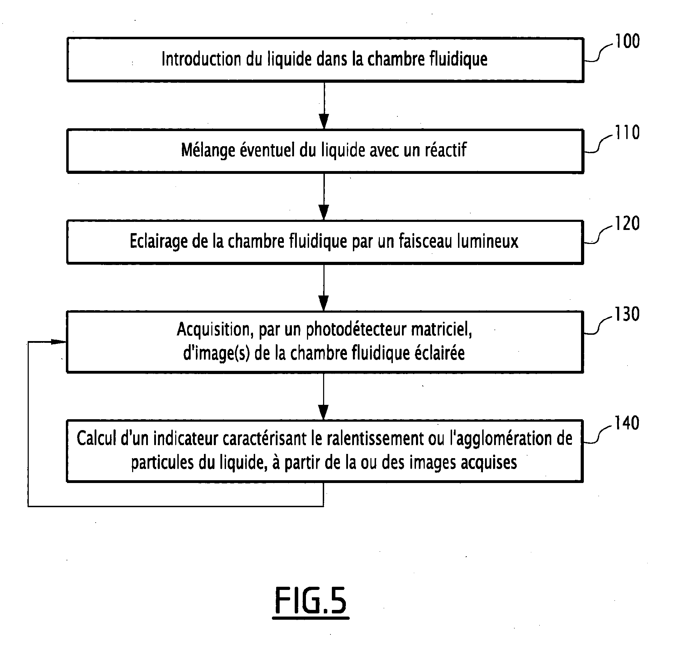

Le fonctionnement du système de caractérisation 10 selon l'invention va être à présent décrit à l'aide la figure 5 représentant un organigramme du procédé de caractérisation selon l'invention.

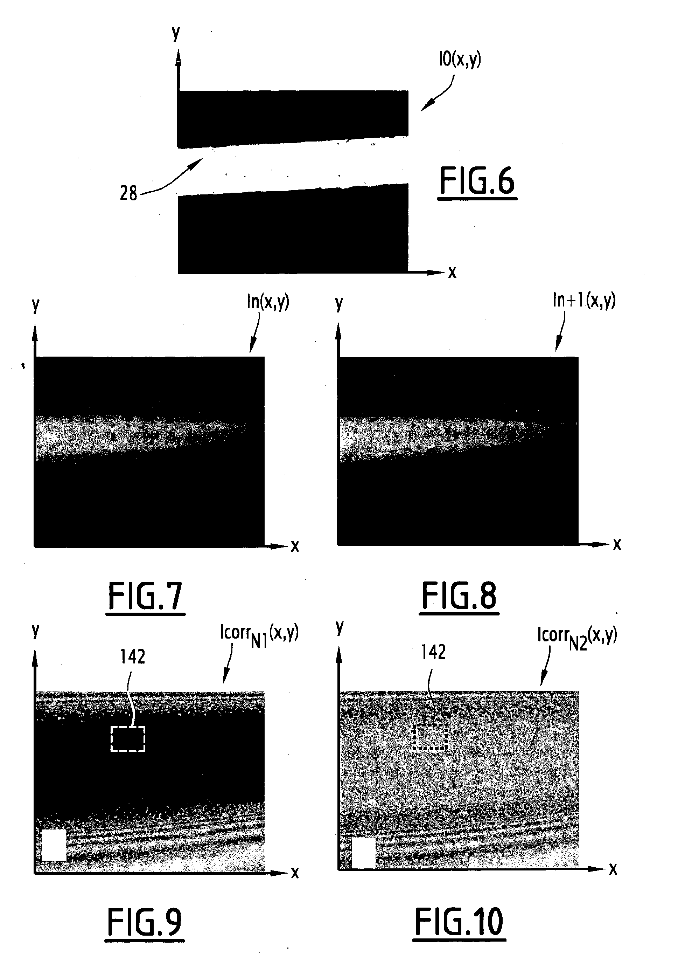

Préalablement à son utilisation, le ou les canaux de circulation 28 de la chambre fluidique sont vides, et une image initiale $I_0$ de la chambre 14 fait alors apparaître une zone blanche correspondant au canal de circulation 28 et des zones délimitant le canal, apparaissant dans cet exemple sous la forme de zones sombres correspondant au reste de la chambre fluidique 14, comme représenté sur la figure 6.

Lors de l'étape initiale 100, le liquide 12 est introduit dans la zone de dépôt 26 de la chambre fluidique. Le liquide 12 s'écoule par capillarité de la zone de dépôt 26 en direction du ou des canaux de circulation 28.

Le liquide 12 est ensuite éventuellement, lors de l'étape 110, mélangé avec un réactif 112, visible sur les figures 3 et 4, et apte à déclencher ou favoriser le phénomène de ralentissement des particules. Le réactif 112 est, par exemple, un réactif lyophilisé apte à favoriser le ralentissement des particules sanguines via une coagulation du sang.

Le réactif 112 est, par exemple, déposé en amont de la zone de détection optique correspondant à la zone à l'intérieur des pointillés sur la figure 3 pour laquelle une image est acquise par le photodétecteur 20. En variante, le réactif 112 est disposé à l'intérieur de la zone de détection optique, comme représenté sur la figure 4. Le mélange entre le liquide 12 et le réactif 112 s'effectue lorsque le liquide 12 s'écoule au contact du réactif 112 à l'intérieur du canal de circulation 28 (flèche F1).

Dans le mode de réalisation décrit, le réactif 112 est une protéine pro-coagulante. Cette protéine est déposée, séchée ou lyophilisée dans le canal de circulation 28. Le réactif 112 est, par exemple, la protéine prothrombine, également appelée TP, lorsque qu'on détermine le paramètre INR (de l'anglais *International Normalized Ratio).*

$$INR = \left(\frac{T}{Tref}\right)^{ISI}$$

T étant le temps de coagulation mesuré, Tref étant le temps de référence considéré, ISI étant un facteur de correction qui dépend des réactifs utilisés pour déclencher la coagulation.

En variante, le réactif 112 est la protéine Ecarine, lorsque le temps de coagulation est mesuré suivant le test ECT (de l'anglais *Ecarin Clotting Time*). En variante, le réactif 112 est la protéine thrombine lorsque le temps de coagulation est mesuré suivant le test TT (de l'anglais *Thrombine Time).*

Le liquide 12 est éclairé par le faisceau lumineux 18 lors de l'étape 120. La source de lumière 16 émet en effet le faisceau lumineux 18 en direction de la chambre fluidique 14 dans laquelle se trouve le liquide 12 selon la direction longitudinale X.

Lors de l'étape 130, le photodétecteur matriciel 20 effectue alors l'acquisition séquentielle de plusieurs images de transmission $I_n(x,y)$, $I_{n+m}(x,y)$ à des instants différents n et n+m. Chaque image de transmission $I_n(x,y)$, $I_{n+m}(x,y)$ est formée par le rayonnement transmis, à l'instant d'acquisition correspondant, par la chambre fluidique 14 éclairée.

Les images $I_n(x,y)$, $I_{n+m}(x,y)$ sont, par exemple, des images immédiatement successives, m étant alors égal à 1, de préférence acquises toutes les secondes, comme représentées sur les figures 7 et 8, où l'écart temporel entre les deux images $I_n(x,y)$, $I_{n+1}(x,y)$ acquises successivement est égal à une seconde.

Les images acquises $I_n(x,y)$, $I_{n+1}(x,y)$ correspondent aux interférences de figures de diffraction engendrées par des particules en suspension dans le liquide 12. L'éclairement des particules par le faisceau 18 spatialement et temporellement cohérent, tel qu'un faisceau laser, engendre une figure de diffraction, qui varie dans le temps à cause du mouvement des particules contenues dans le liquide 12.

L'observation d'une figure de diffraction exploitable, en plaçant le photodétecteur matriciel 20 à une distance aussi faible est notamment due à l'absence d'optique de grossissement entre la chambre fluidique 14 et le photodétecteur 20.

Lors de l'étape d'acquisition 130, le photodétecteur 20 est disposé à une faible distance de la chambre fluidique 14, la deuxième distance D2 entre la chambre fluidique 14 et le photodétecteur 20 selon la direction longitudinale X étant inférieure à 1cm.

A l'issue de l'étape d'acquisition 130, notamment après l'acquisition des images $I_n(x,y)$, $I_{n+1}(x,y)$, le premier logiciel de calcul 36 commence, lors de l'étape 140, par calculer les images $A_n(x,y)$, $A_{n+1}(x,y)$ à l'aide des équations (1), (2) et (3).

Le premier logiciel de calcul 36 calcule ensuite l'image de corrélation $Icorr_{n,n+1}(x,y)$ correspondante à partir des images $A_n(x,y)$, $A_{n+1}(x,y)$ et à l'aide l'équation (4).

Dans le mode de réalisation décrit, les images de corrélation évoluent en fonction du temps, comme représenté sur les figures 9 et 10, où l'image de corrélation $Icorr_{N1,N1+1}(x,y)$, notée $Icorr_{N1}(x,y)$, correspondant à une corrélation entre deux images de transmission $I_n$, $I_{n+1}$ réalisées avec n = N1 avant la coagulation (figure 9) présente un aspect bien différent de l'image $Icorr_{N2}(x,y)$ correspondant à une corrélation entre deux images de transmission $I_{N2}$, $I_{N2+1}$, réalisées avec n = N2 après la coagulation (figure 10).

Le premier logiciel de calcul 36 calcule enfin la valeur du premier indicateur $Ind1_{n,n+1}$ pour chaque image de corrélation $Icorr_{n,n+1}(x,y)$ obtenue. La valeur du premier indicateur $Ind1_{n,n+1}$ est, par exemple, la valeur moyenne des points de l'image de corrélation $Icorr_{n,n+1}(x,y)$ dans la région d'intérêt prédéterminée 142, visible sur les figures 9 et 10. En variante, la valeur du premier indicateur $Ind1_{n,n+1}$ est l'intégrale de l'image dans la région d'intérêt 142 (c'est-à-dire la somme des niveaux de gris de chaque pixel). En variante encore, la valeur du premier indicateur $Ind1_{n,n+1}$ est une fonction de cette intégrale.

En variante, le premier logiciel de calcul 36 commence, lors de l'étape 140, par calculer les images intermédiaires $C_n(x,y)$, $C_{n+1}(x,y)$ à l'aide des équations (5) et (6).

Le premier logiciel de calcul 36 calcule ensuite la valeur du premier indicateur $Ind1_{n,n+1}$ à partir des images intermédiaires $C_n(x,y)$, $C_{n+1}(x,y)$ et à l'aide l'équation (7).

Dans le mode de réalisation décrit, à l'issue de l'étape de calcul 140, le procédé de caractérisation retourne à l'étape 130 afin d'acquérir une nouvelle image de la chambre fluidique 14 éclairée, puis de calculer, de manière analogue lors de l'étape 140, une nouvelle image de corrélation $Icorr_{n+1,n+2}(x,y)$ et une nouvelle valeur du premier indicateur $Ind1_{n+1, n+2}$.

Les étapes d'acquisition 130 et de calcul 140 sont alors réitérées de manière régulière, par exemple toutes les secondes, pendant une durée prédéterminée, par exemple supérieure à 60 secondes, ou jusqu'à un arrêt déclenché par l'utilisateur, notamment au vu de l'évolution temporelle du premier indicateur..

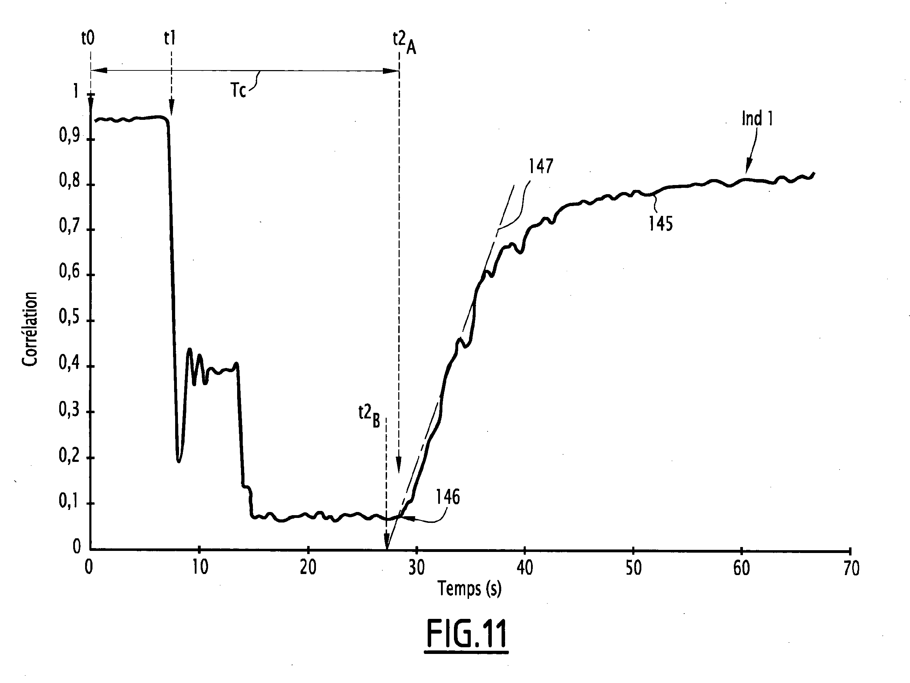

L'évolution temporelle du premier indicateur $Ind1_{n,n+m}$, représentée dans le mode de réalisation décrit par la courbe 145 visible sur la figure 11, permet alors de caractériser la variation de la vitesse des particules contenues dans le liquide 12, tel que le ralentissement des particules sanguines.

Sur la figure 11, un instant initial t0 correspond au mélange du réactif 112 avec le liquide 12, et le canal de circulation 28 en regard de la zone de détection optique est ensuite rempli à un premier instant t1. Autrement dit, l'exemple de réalisation de la figure 11 correspond au cas où le réactif 112 est déposé juste avant la zone de détection optique, comme représenté sur la figure 3.

La courbe 145 montre alors, à partir du premier instant t1, une diminution de la valeur du premier indicateur $Ind1_{n,n+m}$ pour atteindre une valeur minimale inférieure à 0,1. La courbe 145 montre ensuite, à partir d'un deuxième instant $t2_A$, une augmentation rapide de la valeur du premier indicateur $Ind1_{n,n+m}$ jusqu'à une stabilisation de cette valeur autour de 0,8.

La phase entre les premier et deuxième instants t1, $t2_A$, également appelée première phase, au cours de laquelle la valeur du premier indicateur $Ind1_{n,n+m}$ est basse, correspond à une faible corrélation entre les images de transmission acquises successivement. En effet, ceci est dû à un changement important du motif de diffraction d'une image à l'autre au cours de la première phase, à cause du mouvement des particules en suspension dans le liquide 12, dans l'espace correspondant à la région d'intérêt 142.

La phase à compter du deuxième instant $t2_A$ et jusqu'à la fin de la caractérisation, également appelée deuxième phase, correspond quant à elle à un ralentissement des particules dans le liquide 12, ce qui se traduit par une augmentation de la corrélation entre les images successives.

Le logiciel de caractérisation 40, détermine alors, à partir du premier indicateur $Ind1_{n,n+m}$ calculé, l'intervalle temporel entre l'instant origine t0 et l'instant $t2_A$ auquel le premier indicateur présente à nouveau des valeurs de corrélation croissantes. L'intervalle temporel entre l'instant origine t0 et le deuxième instant $t2_A$ est également appelé temps de coagulation Tc. Dans l'exemple de réalisation de la figure 11, le temps de coagulation Tc est égal à environ 29 s.

Le logiciel de caractérisation 40 détermine également la coagulation des particules sanguines à partir du premier indicateur $Ind1_{n,n+m}$ calculé. Le temps de coagulation T correspond par exemple à l'écart temporel entre l'instant initial t0 (t = 0 sur la courbe 145 de la figure 11) et l'instant de coagulation, qui caractérise la coagulation du sang.

Cet instant de coagulation correspond, par exemple, à un point où la courbe 145 atteint un plateau, au-delà duquel les valeurs du premier indicateur $Ind1_{n,n+m}$ n'évoluent pratiquement plus (deuxième instant $t2_A$). On comprend alors que cet instant de coagulation $t2_A$ est également déterminable à partir de la dérivée de la fonction temporelle décrivant l'évolution du premier indicateur $Ind1_{n,n+m}$, par exemple lorsque cette dernière passe en deçà d'un certain seuil.

En variante, cet instant de coagulation est déterminé à partir de la dérivée seconde de cette fonction. A partir de cette dérivée seconde, il est possible de situer un point d'inflexion 146 de la courbe 145. L'instant $t2_A$ correspondant à ce point d'inflexion 146 est alors, par exemple, utilisé pour déterminer l'instant de coagulation.

Dans l'exemple de réalisation de la figure 11, on trace une tangente 147 à la courbe 145, passant par ce point d'inflexion 146. L'instant de coagulation correspond alors à l'abscisse auquel ladite tangente 147 croise la ligne de base de cette courbe (deuxième instant $t2_A$) ou bien à l'abscisse auquel ladite tangente 147 croise l'axe des abscisses (troisième instant $t2_B$). Par ligne de base, on entend la portion de la courbe, sensiblement plane, précédant la brusque remontée de la valeur du premier indicateur $Ind1_{n,n+m}$. Dans l'exemple représenté, la ligne de base correspond à la portion de la courbe 145 dont les abscisses sont comprises environ entre 15 s et 28 s (deuxième instant $t2_A$).

Comme précédemment indiqué, on obtient le temps de coagulation T par une différence entre l'instant de coagulation $t2_A$, $t2_B$ et l'instant initial t0, choisi à l'origine de l'axe des abscisses de la courbe 145.

Le système de caractérisation 10 et le procédé de caractérisation selon l'invention permettent donc de caractériser la variation de la vitesse des particules contenues dans le liquide 12 sur une grande partie du ou des canaux de circulation 28 de la chambre fluidique de par la faible distance entre la chambre fluidique 14 et le photodétecteur 20.

Dans l'exemple de réalisation de la figure 11, le système de caractérisation 10 et le procédé de caractérisation selon l'invention permettent de caractériser un ralentissement des particules par détection d'une augmentation de la corrélation entre les images successives.

L'homme du métier comprendra alors le système de caractérisation 10 et le procédé de caractérisation selon l'invention permettent, de manière analogue, de caractériser une accélération des particules par détection d'une diminution de la corrélation entre les images successives.

La deuxième distance D2 inférieure à 1cm entre la chambre fluidique 14 et le photodétecteur 20 permet également de limiter l'encombrement du système de caractérisation 10.

En outre, l'étendue importante du faisceau lumineux 18 suivant le plan P, c'est-à-dire supérieure à 5mm$^2$, et par exemple comprise entre 5mm$^2$ et 200mm$^2$, permet de limiter l'échauffement du liquide 12 contenu dans la chambre fluidique 14. En effet, la surface importante du faisceau lumineux 18 permet d'avoir une densité optique de puissance faible.

De plus, le fait d'utiliser un faisceau lumineux étendu et de former une image à faible distance de la chambre permet d'examiner un volume de fluide d'autant plus important On limite alors l'influence de phénomènes locaux, susceptibles de devenir prédominants lorsque le faisceau lumineux est plus fin, et le volume de fluide analysé est quasiment ponctuel. L'analyse de la corrélation entre deux images de transmissions $I_n$, $I_{n+m}$ permet de tenir compte de la structure spatiale de la coagulation, dans le plan du canal microfluidique 28. Autrement dit, on observe l'évolution de la coagulation du sang en deux dimensions.

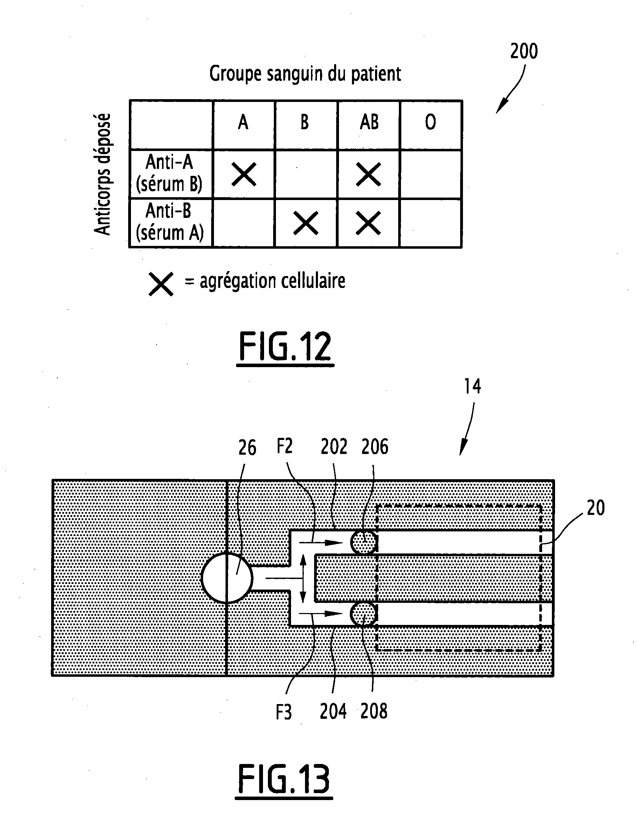

Les figures 12 à 17 illustrent un deuxième mode de réalisation pour lequel les éléments analogues au premier mode de réalisation, décrit précédemment, sont repérés par des références identiques, et ne sont pas décrits à nouveau.

Selon le deuxième mode de réalisation, le système de caractérisation 10 est destiné à caractériser plus particulièrement l'agglomération de particules contenues dans un liquide 12. Le système de caractérisation 10 est, par exemple, apte à caractériser l'agglomération de particules sanguines, telles que des globules rouges, également appelée agglutination de particules sanguines.

Une information relative au groupe sanguin est alors en outre déterminée à partir de l'état d'agglomération, également appelé état d'agglutination.

Comme connu en soi, le groupe sanguin est déterminable selon le test de Beth-Vincent en détectant la présence d'antigènes A ou B impliquant l'absence d'anticorps anti-A ou anti-B. Dans le cas où les hématies du sang testé présentent un antigène A ou B, un complexe antigène-anticorps va se former et conduire à une agrégation cellulaire comme rappelé dans le tableau 200, visible sur la figure 12.

La chambre fluidique 14 comporte deux canaux de circulation 202, 204 distincts, à savoir un premier canal 202 et un deuxième canal 204, comme représenté sur la figure 13.

Selon le deuxième mode de réalisation, la source de lumière 16 est tout type de source de lumière. La source de lumière 16 n'est pas nécessairement spatialement et temporellement cohérente.

Selon le deuxième mode de réalisation, le deuxième logiciel de calcul 38 est propre à calculer le deuxième indicateur Ind2 apte à caractériser l'agglomération des particules, le deuxième indicateur Ind2 étant un indicateur d'intensité pour chaque image acquise $I_n(x,y)$. Le deuxième indicateur Ind2 est représentatif de l'histogramme de l'intensité de chaque pixel dans l'image $I_n$, ou dans une région d'intérêt de cette dernière. On le détermine, par exemple, en mesurant l'intensité totale de l'image $I_n$ ou dans une région d'intérêt prédéterminée de l'image $I_n$, éventuellement après seuillage.

Le logiciel de caractérisation 40 est propre à déterminer ensuite un état d'agglomération des particules du liquide 12 à partir du deuxième indicateur calculé Ind2. L'état d'agglomération est, par exemple, déterminé lorsque le deuxième indicateur Ind2 dépasse un seuil prédéterminé.

Dans le mode de réalisation décrit, où le liquide 12 contient du sang, les particules sont, par exemple, des globules rouges, et le logiciel de caractérisation est alors propre à déterminer une information relative au groupe sanguin à partir de l'état d'agglomération.

Le fonctionnement de ce deuxième mode de réalisation va être à présent décrit à l'aide des figures 13 à 17.

Lors de l'étape initiale 100, le liquide 12, par exemple l'échantillon sanguin d'un donneur dont on veut déterminer le groupe sanguin, est introduit dans la zone de dépôt 26 de la chambre fluidique. Le liquide 12 s'écoule alors de la zone de dépôt 26 en direction des canaux de circulation 202, 204, par exemple par capillarité.

Le liquide 12 est ensuite mélangé avec un premier 206 et un deuxième 208 réactifs distincts, lors de l'étape 110, comme représenté sur la figure 13.

Chaque réactif 206, 208 est, par exemple, déposé en amont de la zone de détection optique correspondant à la zone à l'intérieur des pointillés sur la figure 13 pour laquelle une image est acquise par le photodétecteur 20.

Le mélange entre le liquide 12 et les premier et deuxième réactifs 206, 208 s'effectue lorsque le liquide 12 s'écoule au contact du premier réactif 206 à l'intérieur du premier canal 202 (flèche F2), et respectivement du deuxième réactif 208 à l'intérieur du deuxième canal 204 (flèche F3).

Dans le mode de réalisation décrit, le premier réactif 206 est un sérum de donneur A, c'est-à-dire contenant des anticorps anti-B, et le deuxième réactif 208 est un sérum de donneur B, c'est-à-dire contenant des anticorps anti-A.

En fonction du groupe sanguin associé à l'échantillon sanguin 12, une agrégation cellulaire va alors se produire ou non dans chacun des canaux de circulation 202, 204.

Le liquide 12, tel que l'échantillon sanguin mélangé aux premier et deuxième réactifs 206, 208, est ensuite éclairé par le faisceau lumineux 18 lors de l'étape 120.

Lors de l'étape 130, le photodétecteur matriciel 20 effectue alors l'acquisition d'une image de transmission $I(x,y)$ correspondant à une zone de détection optique englobant les deux canaux de circulation 202, 204.

L'homme du métier observera que, selon le deuxième mode de réalisation, l'acquisition d'une seule image de transmission $I(x,y)$ permet de caractériser l'agglomération des particules contenues dans le liquide 12, en comparant cette image à une image de référence $I_{ref}(x,y)$, cette dernière étant par exemple une image réalisée sur une zone de référence, non représentée, dans laquelle le sang n'est pas mélangé à un réactif. Cette zone de référence est, par exemple, un troisième canal, de géométrie identique à celle du premier ou deuxième canal 202, 204, et ne comportant aucun réactif.

En variante, il s'agit d'une zone située sur le premier canal 202 ou sur le deuxième canal 204, en amont du réactif 206, 208.

En variante, l'image de référence $I_{ref}(x,y)$ est réalisée au même endroit que l'image de transmission, juste après le remplissage du canal par le liquide analysé, l'image de transmission $I(x,y)$ étant réalisée, dans les mêmes conditions, après un certain temps, par exemple 1 minute, de telle sorte que l'effet éventuel du réactif sur le liquide analysé soit mesurable.

L'image acquise $I(x,y)$ correspond de manière analogue à la diffraction et à la diffusion du faisceau lumineux 18 par les particules en suspension dans le liquide 12. De préférence, cette image est réalisée dans des conditions identiques pour les deux canaux, ainsi que pour la zone de référence. Par conditions identiques, on entend notamment les conditions d'éclairement, la distance source-détecteur, les caractéristiques du détecteur utilisé, le temps de pose, le champ observé, la taille de l'image.

L'éclairement des particules par le faisceau lumineux 18 engendre un motif de diffraction. Comme précédemment indiqué, l'absence de lentille de grossissement entre le fluide et le photodétecteur 20, couplée à la surface importante du faisceau incident, permet de former une image exploitable à faible distance, couvrant un champ important de fluide, tel qu'un champ présentant une aire de plusieurs $mm^2$.

Lors de l'étape d'acquisition 130, le photodétecteur 20 est disposé à proximité de la chambre fluidique 14, la deuxième distance D2 entre la chambre fluidique 14 et le photodétecteur 20 selon la direction longitudinale X étant inférieure à 1cm.

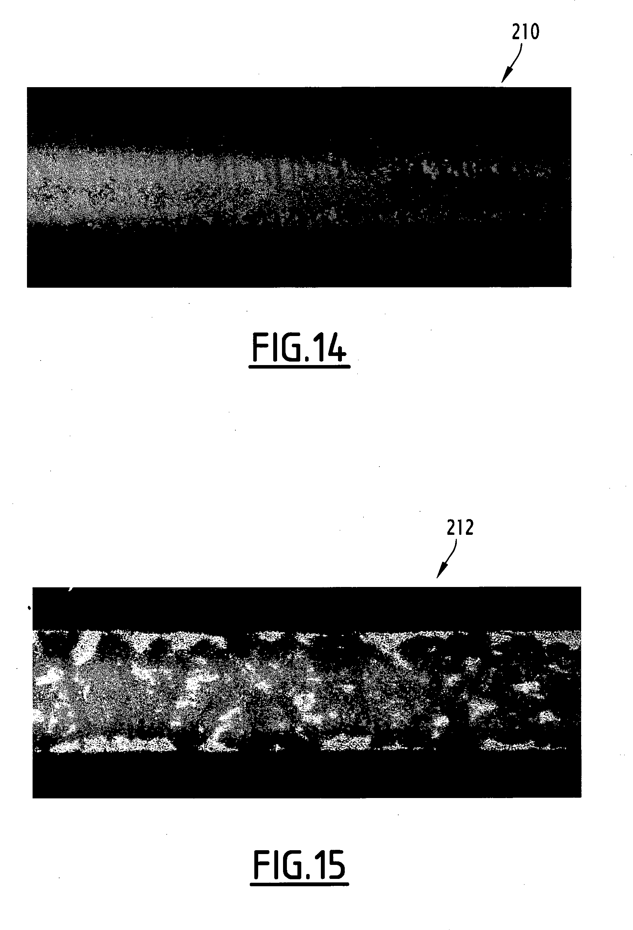

Dans l'exemple de réalisation des figures 14 et 15, représentant une image 210 acquise du premier canal 202, et respectivement une image 212 acquise du deuxième canal 204, une agrégation cellulaire est observée seulement dans le deuxième canal 204 de par la présence de tâches blanches dans l'image 212. Autrement dit, le groupe sanguin associé à l'échantillon sanguin testé est le groupe B d'après le tableau 200 de la figure 12.

A l'issue de l'étape d'acquisition 130, le deuxième logiciel de calcul 38 calcule, lors de l'étape 140, le deuxième indicateur Ind2 apte à caractériser l'agglomération des particules, le deuxième indicateur Ind2 étant un indicateur d'intensité pour chaque image acquise I(x,y). Le deuxième indicateur Ind2 est représentatif de l'intensité dans la région d'intérêt prédéterminée de l'image, en particulier de la distribution de l'intensité des pixels dans ladite région.

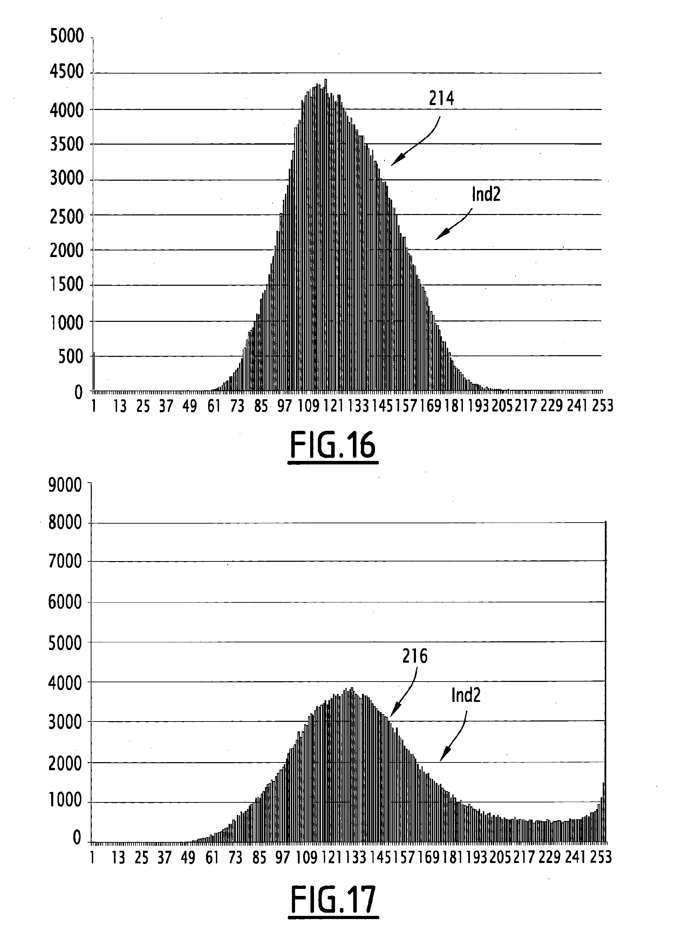

Le deuxième indicateur Ind2 est, par exemple, une caractéristique de l'image, et en particulier de l'histogramme du niveau de gris de l'image acquise pour chaque canal 202, 204, et le cas échéant, du canal de référence comme illustré sur les figures 16 et 17, représentant un histogramme 214 du niveau de gris de l'image acquise du premier canal 202, et respectivement un histogramme 216 du niveau de gris de l'image acquise du deuxième canal 204. Chaque histogramme de niveaux de gris 214, 216 présente en abscisse les valeurs de niveaux de gris et en ordonnée la population de pixels, c'est-à-dire le nombre de pixels, pour un niveau de gris donné en abscisse. Une caractéristique de l'histogramme est, par exemple, l'intensité moyenne des pixels, notée $I_{mean}$, après un éventuel seuillage de l'image, ce seuillage permettant de ne conserver que l'information des pixels dont l'intensité est supérieure à un certain seuil.

En se référant à l'exemple représenté sur les figures 16 et 17, après avoir effectué un seuillage à l'intensité correspondant à la valeur 120, on comprend que l'intensité moyenne de l'image correspondant à la figure 17, est supérieure à l'intensité moyenne de l'image correspondant à la figure 16. Cela vient du fait que l'histogramme de la figure 17, représentant l'observation d'une agglutination de particules, comprend davantage de pixels intenses (niveaux de gris supérieurs à 200) que l'histogramme de la figure 16, ce dernier représentant l'observation d'une non agglutination. Le deuxième indicateur Ind2 est alors, par exemple, établi selon l'intensité moyenne de l'image.

Selon une variante, on détermine, pour chaque image réalisée, l'intensité $I_{max}$, cette dernière correspondant, sur l'histogramme de l'image, à la valeur la plus élevée de l'intensité rassemblant un nombre prédéterminé de pixels, par exemple 500 pixels. On détermine ensuite l'écart entre $I_{max}$ et $I_{mean}$, par la soustraction $I_{max}$-$I_{mean}$, le deuxième indicateur Ind2 représentant alors cet écart. Sur l'histogramme de la figure 17, le deuxième indicateur Ind2 ainsi défini est plus élevé que sur l'histogramme de la figure 16. La valeur du deuxième indicateur Ind2 ainsi déterminée permet de conclure à la présence ou à l'absence d'un phénomène d'agglutination, via une comparaison, par exemple avec une valeur $Ind2_{ref}$ obtenue sur une zone de référence, ou encore avec une valeur prédéterminée, la prédétermination de cette valeur étant par exemple effectuée selon des essais expérimentaux.

Selon une variante, sur chaque image de transmission, on détermine l'intensité $I_{peak}$ correspondant à la valeur maximale de l'histogramme, c'est-à-dire la valeur d'intensité rassemblant le nombre le plus élevé de pixels. Sur les figures 16 et 17, cette valeur correspond au pic de chaque distribution, respectivement égales à 120 et 130. On détermine également la valeur maximum $I_{max}$ rassemblant un nombre de pixels de valeur supérieure à un seuil prédéterminé. En se référant aux figures 16 et 17, et en adoptant un seuil de 500, $I_{max}$ est respectivement égale à 181 et 256. Le deuxième indicateur Ind2 correspond à la distance entre $I_{max}$ et $I_{peak}$, respectivement 61 pour la figure 16 et 126 pour la figure 17. On conclut à une agglutination lorsque le deuxième indicateur Ind2 est supérieur, par exemple de 25%, à un certain seuil prédéterminé, ou lorsqu'il est supérieur à l'indicateur $Ind2_{ref}$ établi pour la zone de référence.

Selon une variante, le deuxième indicateur Ind2 est un indicateur de comparaison entre une région d'intérêt d'une image de transmission I et une image de référence $I_{ref}$ ne contenant pas de réactif (et donc dans lequel l'agglutination ne se produit pas) comme ci-dessous :

$$Ind2 = \frac{\sum_{x}\sum_{y}\left|I(x,y) - I_{ref}(x,y)\right|}{\sum_{x}\sum_{y}I(x,y) + I_{ref}(x,y)}$$

On compare le deuxième indicateur Ind2avec un seuil prédéterminé, par exemple 0,25. Ainsi, si le deuxième indicateur Ind2 est supérieur à ce seuil; on conclut à une agglutination.

Le logiciel de caractérisation 40 est propre à déterminer ensuite un état d'agglomération des particules du liquide 12 à partir du deuxième indicateur calculé Ind2.

L'état d'agglomération est, par exemple, déterminé lorsque le deuxième indicateur Ind2 dépasse un seuil prédéterminé.

Si la comparaison est positive, c'est-à-dire si le niveau de gris obtenu est supérieur au seuil prédéterminé, alors le logiciel de caractérisation 40 en déduit la présence d'une agrégation cellulaire dans le canal 202, 204 correspondant.

Dans le deuxième mode de réalisation décrit, le logiciel de caractérisation 40 détermine enfin le groupe sanguin associé à l'échantillon sanguin 12 testé à partir du type des premier et deuxième réactifs 206, 208, ainsi que du tableau 200.

Les avantages de ce deuxième mode de réalisation sont identiques à ceux du premier mode de réalisation décrit précédemment.

En complément du premier mode de réalisation, la chambre fluidique 14 comporte une pluralité de canaux, par exemple les deux canaux 202, 204 visibles sur la figure 13, afin de caractériser la variation de la vitesse des particules du liquide 12 lorsque le liquide 12 est mélangé avec des réactifs différents, un réactif respectif étant alors disposé dans chaque canal 202, 204 de la chambre fluidique 14. Cela permet, avec un même dispositif, de déterminer différents paramètres d'analyse d'un même échantillon liquide, par exemple le temps de coagulation et le groupe sanguin.

Une telle chambre fluidique 14 est, par exemple, avantageuse pour caractériser la coagulation du liquide 12 contenant du sang, avec différents réactifs aptes à favoriser le ralentissement des particules sanguines via une coagulation du sang, tels que les différents réactifs 112 définis ci-dessus.

On conçoit ainsi que le système de caractérisation 10 selon l'invention permet d'observer une plus grande partie de la chambre fluidique 14, tout en ayant un encombrement limité.

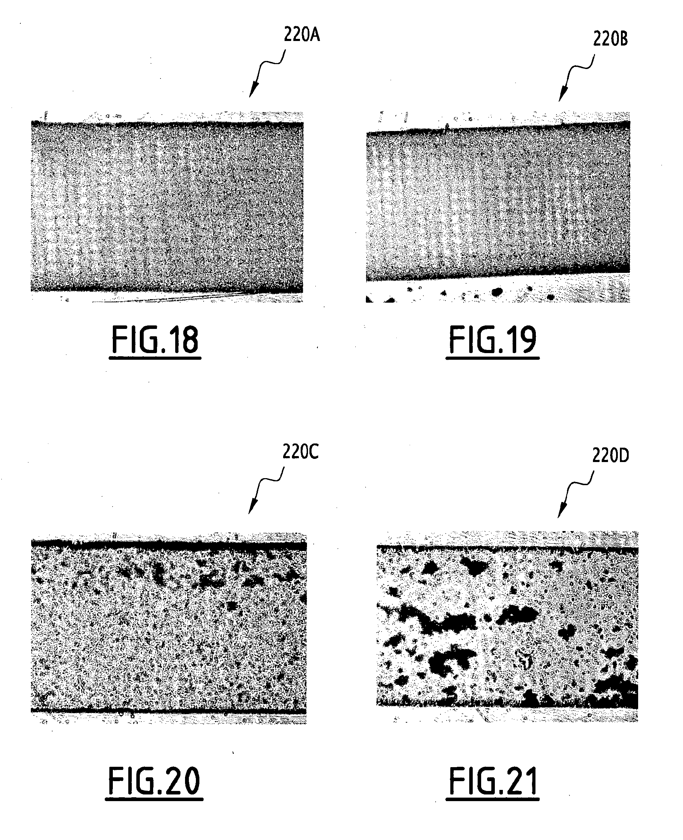

Les figures 18 à 29 illustrent un deuxième exemple du deuxième mode de réalisation, dans lequel le liquide à caractériser 12 est un liquide biologique, en particulier du sang ou du sang dilué, et le système de caractérisation 10 est apte à caractériser l'agglomération de particules, en l'occurrence des globules rouges dans le liquide biologique 12.

Le liquide à caractériser 12 comporte, dans cet exemple, du sang dilué au 1/20 dans un tampon PBS (de l'anglais *Phosphate Buffered Saline*), le tampon comportant 1% en volume de FBS (de l'anglais *Foetal Bovine Serum*).

Le volume de sang dilué est de 40 $\mu$l, auquel on ajoute une quantité variable d'anticorps, tel qu'un anti-globule rouge appelé CD235A, commercialisé à titre d'exemple par la société Becton Dickinson sous la référence BD 555569. La quantité d'anticorps ajoutée varie de 0 à 1 $\mu$g d'anticorps par $\mu$l de sang non dilué, ce qui correspond à une concentration comprise entre 0 et 6.7 $\mu$M..

L'ajout de cet anticorps permet de masquer les antigènes de surface des globules rouges (en particulier la glycophorine A), ce qui entraîne leur agglutination.

L'objectif de ce deuxième exemple est de montrer qu'il est possible de caractériser un état d'agglutination de particules sanguines, par exemple de globules rouges, par imagerie sans lentille à l'aide du système de caractérisation 10.

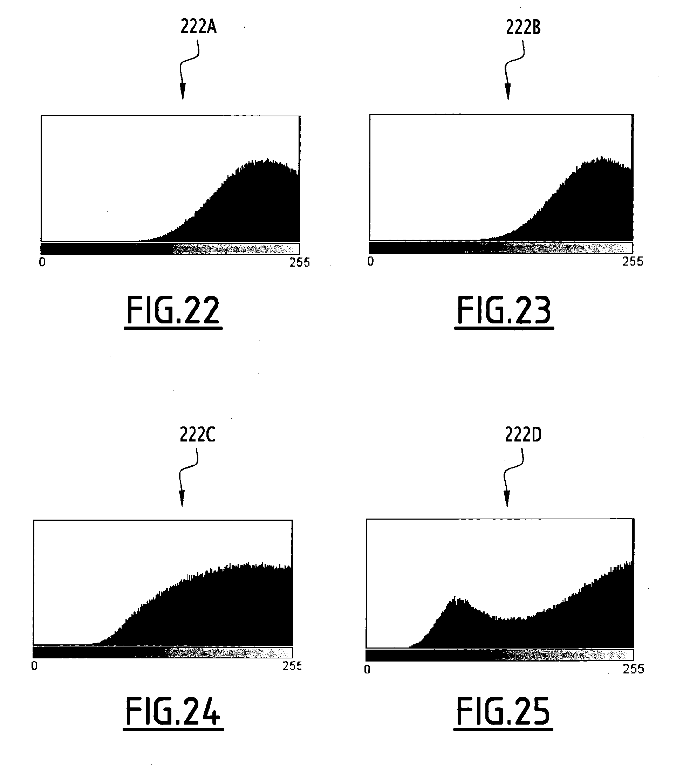

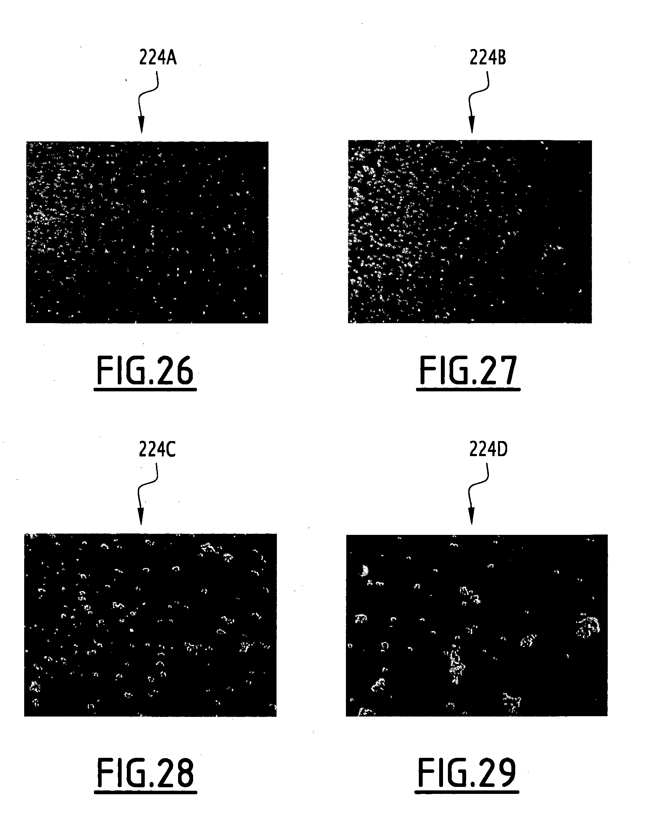

A chaque quantité d'anticorps ajoutée dans le liquide à caractériser 12, on effectue une acquisition de l'échantillon de liquide à caractériser 12 à l'aide du système de caractérisation 10, c'est-à-dire par imagerie sans lentille, les images 220A, 220B, 220C et 220D obtenues étant visibles sur les figures 18 à 21. Un histogramme en niveau de gris de l'intensité des pixels de chacune ces images 220A, 220B, 220C et 220D est ensuite calculé, les histogrammes calculés 222A, 222B, 222C et 222D étant visibles sur les figures 22 à 25. Des images de référence 224A, 224B, 224C et 224D de l'échantillon de liquide à caractériser 12 sont également obtenues avec un microscope, comme représenté sur les figures 26 à 29. Il est à noter que, pour l'observation en microscopie, l'échantillon sanguin est dilué avec un facteur de dilution d'un dixième.

Dans ce deuxième exemple, la source de lumière 16 est une diode Laser, présentant un spectre d'émission centré sur une longueur d'onde $\lambda$ par exemple égale à 670 nm, et la première distance D1 est sensiblement égale à 8 cm. L'échantillon est confiné dans la chambre fluidique 14 comportant un canal 28 d'épaisseur 150 $\mu$m ménagé entre deux parois transparentes d'épaisseur 200 $\mu$m. Ces parois sont réalisées en matériau plastique, par exemple en matériau COP (de l'anglais *Cyclo Olefin Polymer*).

La chambre fluidique 14 est directement posée sur le capot de verre du photodétecteur matriciel 20, tel qu'un capteur CMOS, comportant 1280 * 1024 pixels, chaque pixel étant de dimension 5 $\mu$m x 5 $\mu$m, de telle sorte que la chambre fluidique 14 est disposée entre le capteur CMOS et la source de lumière 16. La deuxième distance D2 est alors de préférence inférieure à 1 cm, par exemple égale à 550 $\mu$m.

Les acquisitions d'images sont par exemple réalisées avec un temps de pose 5 ms, avec une image par acquisition. Les images 220A, 220B, 220C et 220D correspondent respectivement à une quantité d'anticorps ajoutée croissante. Plus précisément, les images 220A, 220B, 220C et 220D correspondent respectivement à :

- une quantité d'anticorps sensiblement nulle,
- une quantité d'anticorps inférieure à une concentration seuil C,
- une quantité d'anticorps égale à la concentration seuil C, et
- une quantité d'anticorps égale à 2 fois la concentration seuil C.

Lorsque la quantité d'anticorps ajoutée dépasse la concentration seuil C, les globules rouges s'agglomèrent et les images obtenues par imagerie sans lentille à l'aide du système de caractérisation 10 reflètent la taille des agglutinats. La valeur de la concentration seuil C est, par exemple, égale à 250 ng d'anticorps pour 1 $\mu$l de sang non dilué, ce qui correspond à 1,7 $\mu$M.

On observe que l'agglomération de globules rouges entraîne l'apparition de zones étendues claires (fort niveau de gris) délimitées par des zones sombres (faible niveau de gris). Cet effet de segmentation d'image selon des zones comportant plusieurs dizaines, voire centaines de pixels, de niveaux de gris comparables, peut être observée en comparant les images 220A (figure 18) ou 220B (figure 19), sur lesquelles aucune agglutination n'est observée, aux images 220C (figure 20) et 220D (figure 21), sur lesquelles on observe une agglutination. La présence ou l'absence d'une agglutination de particules observable sur les images 220A, 220B, 220C et 220D est confirmée par les observations au microscope représentées sur les images 224A (figure 26), 224B (figure 27), 224C (figure 28) et 224D (figure 29) respectivement. Cela se traduit par une évolution de l'histogramme de chaque image, ce dernier tendant à s'étirer vers les faibles valeurs de niveau de gris au fur et à mesure que la quantité d'agglomérats augmente, comme cela est visible depuis l'histogramme 222A correspondant à l'image 220A en allant vers l'histogramme 222D correspondant à l'image 220D.

L'état d'agglutination de l'échantillon sanguin est alors quantifié par le calcul du deuxième indicateur Ind2 selon plusieurs variantes possibles :

- le deuxième indicateur Ind2 est, selon une première variante, égal à l'écart-type de la distribution en intensité des pixels de la zone d'intérêt examinée, et est alors noté $Ind2_A$,
- le deuxième indicateur Ind2 est, selon une deuxième variante, égal au nombre de pixels en deçà d'un certain seuil, ce seuil étant par exemple une fraction du niveau de gris maximal, divisé par le nombre total de pixels dans la zone d'intérêt examinée, et le deuxième indicateur Ind2 calculé selon cette deuxième variante est alors noté $Ind2_B$. Dans l'exemple des figures 22 à 25, la valeur du seuil est égale à 125.

Le tableau 1, ci-dessous, présente la valeur du deuxième indicateur Ind2 selon ces deux variantes et pour chacune des zones d'intérêt représentées sur les figures 18 à 21.

| Figures | 18 | 19 | 20 | 21 |
|---|---|---|---|---|
| $Ind2_A$ | 36 | 33 | 51 | 59 |
| $Ind2_B$ | $6.5 \cdot 10^{-3}$ | $6.5 \cdot 10^{-3}$ | $8.7 \cdot 10^{-2}$ | $1.4 \cdot 10^{-1}$ |

Lorsque le deuxième indicateur $Ind2_A$ selon la première variante est inférieur à une valeur seuil, comprise par exemple entre 40 et 45, il n'y a pas d'agglutination observable. Au-delà de cette valeur seuil, plus la valeur du deuxième indicateur $Ind2_A$ est élevée, plus la quantité de particules agglutinées est importante.

Le deuxième indicateur $Ind2_B$, calculé selon la deuxième variante, permet d'aboutir aux mêmes conclusions, en prenant une valeur seuil comprise entre $1 \cdot 10^{-2}$ et $5 \cdot 10^{-2}$.

Ainsi, on constate qu'il est possible d'observer, voire de quantifier, un état d'agglutination de particules dans le liquide biologique 12, à l'aide d'un indicateur calculé à partir d'une image obtenue par le système de caractérisation 10, c'est-à-dire par imagerie sans lentille, et notamment le deuxième indicateur $Ind2_A$, $Ind2_B$, selon les première et deuxième variantes de ce deuxième exemple, ce deuxième indicateur étant une fonction de la distribution de l'intensité des pixels des images 220A, 220B, 220C et 220D acquises par le système de caractérisation 10.

L'utilisation du système de caractérisation 10 est également envisageable dans un test diagnostic basé sur la détection d'agglutinats dans un fluide biologique.

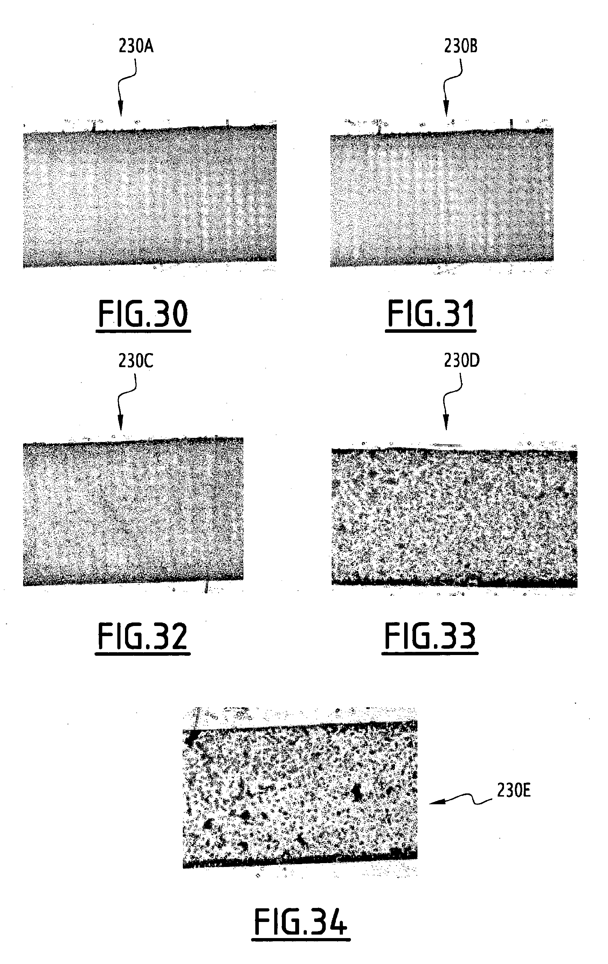

Les figures 30 à 45 illustrent un troisième exemple du deuxième mode de réalisation, dans lequel le liquide à caractériser 12 est un liquide biologique, en particulier du sang ou du sang dilué, et le système de caractérisation 10 est apte à caractériser l'agglomération, également appelé agglutination, de particules dans le liquide biologique 12.

Dans ce troisième exemple, on met en évidence la détection de l'agglutination de globules rouges dans un échantillon sanguin, comportant une quantité variable de protéine A, l'agglutination étant provoquée par l'ajout d'une quantité donnée d'un réactif (un anticorps).

Le liquide à caractériser 12 comporte, par exemple, du sang dilué au 1/20 dans un tampon PBS (de l'anglais *Phosphate Buffered Saline*), le tampon comportant 1% en volume de FBS (de l'anglais *Foetal Bovine Serum*).

Le volume de sang dilué est de 40 µl, auquel on incube un anticorps, tel qu'un anti-globule rouge appelé CD235A, commercialisé à titre d'exemple par la société Becton Dickinson sous la référence BD 555569, avec une solution de protéine A en quantité variable. La durée d'incubation est de 1 heure.

Ainsi, on dispose de plusieurs solutions, dites anticorps - protéine A, dans lesquelles le ratio molaire anticorps - protéine A est variable. Ces solutions sont susceptibles d'entraîner l'agglutination de globules rouges, d'où la dénomination « solutions pro-agglutinantes ». Un volume d'1,2 µl de chacune de ces solutions est incubée avec 40 µl d'échantillon de sang dilué décrit ci-dessus, pendant 1,5 heures, chacun de ces mélanges formant un échantillon de liquide à caractériser 12.

Dans chacun des mélanges ainsi obtenu, la concentration molaire d'anticorps S est inférieure au seuil C déterminé dans le deuxième exemple précédent. Autrement dit, cette concentration d'anticorps ne permet pas l'agglutination spontanée de globules rouges. En l'occurrence, cette concentration S est de 100 ng d'anticorps par µl de sang non dilué, soit 0.7 µM.

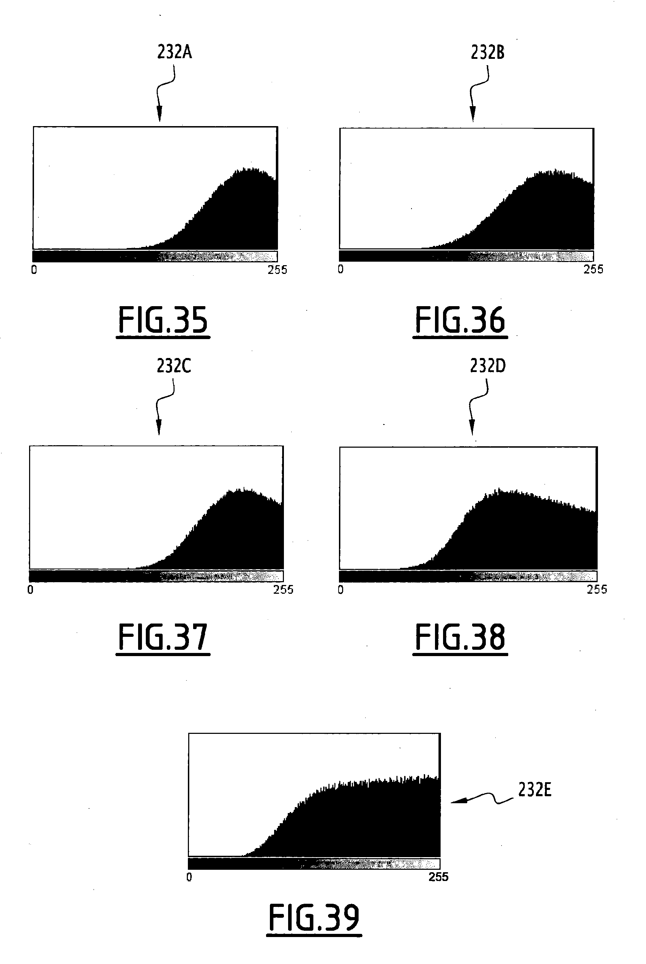

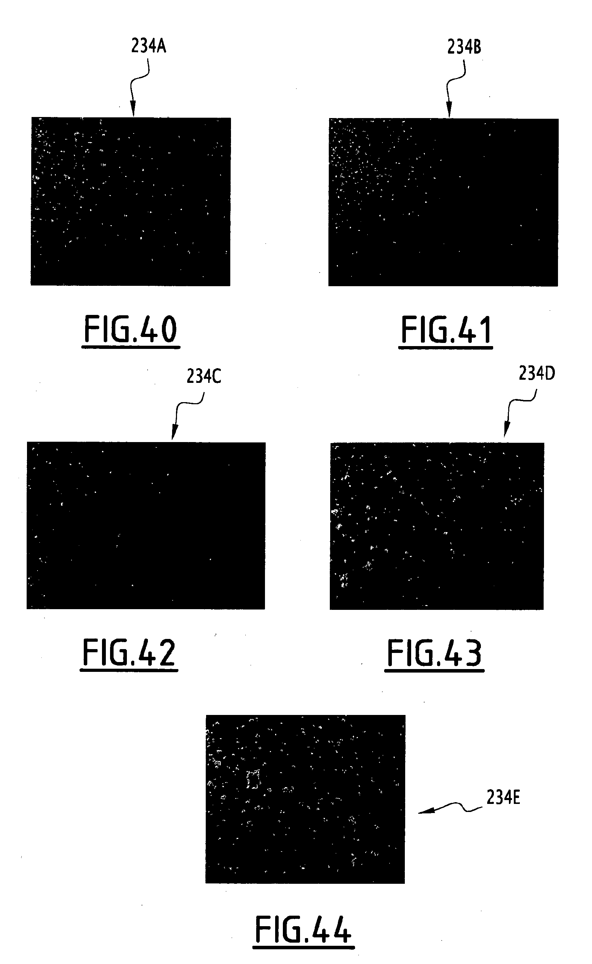

Pour chacun des échantillons de liquide à caractériser 12, on effectue une acquisition d'image à l'aide du système de caractérisation 10, c'est-à-dire par imagerie sans lentille, les images acquises 230A, 230B, 230C, 230D et 230E obtenues étant visibles sur les figures 30 à 34. Un histogramme en niveau de gris de l'intensité des pixels de chacune ces images 230A, 230B, 230C, 230D et 230E est ensuite calculé, les histogrammes calculés 232A, 232B, 232C, 232D et 232E étant visibles sur les figures 35 à 39. Des images de référence 234A, 234B, 234C, 234D et 234E de chacun des échantillons de liquide à caractériser 12 sont également obtenues avec un microscope, comme représenté sur les figures 40 à 44. Il est à noter que, pour l'observation en microscopie, l'échantillon sanguin est dilué avec un facteur de dilution d'un dixième.

Dans ce troisième exemple, la source de lumière 16 est une diode Laser, présentant un spectre d'émission centré sur une longueur d'onde λ égale à 670 nm, et la première distance D1 est sensiblement égale à 8 cm. L'échantillon est confiné dans la chambre fluidique 14 comportant un canal 28 d'épaisseur 150 μm ménagé entre deux parois transparentes d'épaisseur 200 μm. Ces parois sont réalisées en matériau plastique, par exemple en matériau COP (de l'anglais *Cyclo Olefin Polymer*).

La chambre fluidique 14 est directement posée sur le capot de verre du photodétecteur matriciel 20, tel qu'un capteur CMOS. Le capteur CMOS est par exemple une matrice 1280 par 1024 pixels, chaque pixel étant en forme d'un carré de 5 μm de côté ; de telle sorte que la chambre fluidique 14 est disposée entre le capteur CMOS et la source de lumière 16. La deuxième distance D2 est alors de préférence inférieure à 1 cm, par exemple égale à 550 μm.

Les acquisitions d'images sont par exemple réalisées avec un temps de pose 5 ms, avec une image par acquisition. Les images 230B, 230C, 230D et 230E correspondent respectivement à une quantité de protéine A ajoutée croissante. Plus précisément, les images 230A, 230B, 230C, 230D et 230E correspondent respectivement à :

- absence d'anticorps, soit un ratio molaire Anticorps : Protéine A = 0 : 40 (0 molécule d'anticorps pour 40 molécules de protéine A),
- absence de protéine A, soit un ratio molaire Anticorps - Protéine A = 1 : 0 (1 molécule d'anticorps pour 0 molécule de protéine A),
- ratio molaire Anticorps : Protéine A = 1 : 1 (1 molécule d'anticorps pour 1 molécule de protéine A),
- ratio molaire Anticorps : Protéine A = 1 : 5 (1 molécule d'anticorps pour 5 molécules de protéine A), et
- ratio molaire Anticorps : Protéine A = 1 : 40 (1 molécule d'anticorps pour 40 molécules de protéine A).

L'anticorps sert ici d'agent de couplage entre une molécule de protéine A et un globule rouge, comme cela sera détaillé ultérieurement.

En présence de protéine A et en l'absence d'anticorps, aucune agglutination de globules rouges n'est observée, comme représenté sur la figure 30. En présence d'anticorps et en l'absence de protéine A, aucune agglutination de globules rouges n'est observée, ceci étant visible sur la figure 31.

Lorsque le ratio anticorps : protéine A est égal à 1 : 1, aucune agglutination de globules rouges n'est également observée, comme représente sur la figure 32.

Lorsque le ratio anticorps : protéine A est égal à 1 : 5, on observe une agglutination de globules rouges, visible sur la figure 33. Lorsque le ratio anticorps : protéine A est égal à 1 : 40, on observe également une agglutination de globules rouges, visible sur la figure 34, la taille des agglutinats observés sur la figure 34 étant supérieure à celle des agglutinats observés sur la figure 33.

Les figures 35 à 39 représentent l'histogramme de l'intensité des pixels des figures 30 à 34 respectivement.

On observe que l'agglomération de globules rouges entraîne l'apparition de zones claires (fort niveau de gris) délimitées par une zone sombre (faible niveau de gris). Cet effet de segmentation d'image selon des zones comportant plusieurs dizaines, voire centaines de pixels, de niveaux de gris comparables, visible en comparant les images 230A (figure 30) ou 230B (figure 31) ou 230C (figure 32), sur lesquelles aucune agglutination n'est observée, aux images 230D (figure 33) et 230E (figure 34), sur lesquelles on observe une agglutination. La présence ou l'absence d'une agglutination de particules observable sur les images 230A, 230B, 230C, 230D et 230E est confirmée par les observations au microscope représentées sur les images 234A (figure 40), 234B (figure 41), 234C (figure 42), 234D (figure 43) et 234E (figure 44) respectivement. Cela se traduit par une évolution de l'histogramme de chaque image, ce dernier tendant à s'étirer vers les faibles valeurs de niveau de gris au fur et à mesure que la quantité d'agglomérats augmente, comme cela est visible depuis l'histogramme 232A correspondant à l'image 230A en allant vers l'histogramme 232E correspondant à l'image 230E. L'état d'agglutination de l'échantillon sanguin est alors quantifié par le calcul du deuxième indicateur Ind2 selon plusieurs variantes possibles :

- le deuxième indicateur Ind2 est, selon une première variante, égal à l'écart-type de la distribution en intensité des pixels de la zone d'intérêt examinée, et est alors noté $Ind2_A$,
- le deuxième indicateur Ind2 est, selon une deuxième variante, égal au nombre de pixels en deçà d'un certain seuil, ce seuil étant par exemple une fraction du niveau de gris maximal, divisé par le nombre total de pixels dans la zone d'intérêt examinée, et le deuxième indicateur Ind2 calculé selon cette deuxième variante est alors noté $Ind2_B$. Dans l'exemple des figures 22 à 25, la valeur du seuil est par exemple égale à 125.

Le tableau 2, ci-dessous, présente la valeur du deuxième indicateur Ind2 selon ces deux variantes et pour chacune des zones d'intérêt représentées sur les figures 30 à 34.

| Figures | 30 | 31 | 32 | 33 | 34 |
|---|---|---|---|---|---|
| $Ind2_A$ | 33 | 39 | 35 | 50 | 52 |
| $Ind2_B$ | $4.0\ 10^{-3}$ | $1.5\ 10^{-2}$ | $5.5\ 10^{-3}$ | $6.5\ 10^{-2}$ | $9.5\ 10^{-2}$ |

Lorsque le deuxième indicateur $Ind2_A$ selon la première variante est inférieur à une valeur seuil, comprise par exemple entre 40 et 45, il n'y a pas d'agglutination observable. Au-delà de cette valeur seuil, plus la valeur du deuxième indicateur $Ind2_A$ est élevée, plus la quantité de particules agglutinées est importante.

Le deuxième indicateur $Ind2_B$, calculé selon la deuxième variante, permet d'aboutir aux mêmes conclusions, en prenant une valeur seuil comprise entre $1\ 10^{-2}$ et $5\ 10^{-2}$.

Ainsi, on constate qu'il est possible d'observer, voire de quantifier, un état d'agglutination de particules dans le liquide biologique 12, à l'aide d'un indicateur calculé à partir d'une image obtenue par le système de caractérisation 10, c'est-à-dire par imagerie sans lentille, et notamment le deuxième indicateur $Ind2_A$, $Ind2_B$, selon les première et deuxième variantes de ce deuxième exemple, qui est fonction de la distribution de l'intensité des pixels des images 230A, 230B, 230C, 230D et 230E acquises par le système de caractérisation 10.

En outre, plus la quantité de protéine A est importante, plus la taille des agglutinats est importante, la quantité d'anticorps ajoutée étant constante. Ainsi, le deuxième indicateur $Ind2_A$, $Ind2_B$ quantifiant l'état d'agglutination permet également de quantifier une quantité de protéine dans l'échantillon sanguin.

En fonction du ratio molaire anticorps-protéine A, les globules rouges s'agglomèrent et les images 230A, 230B, 230C, 230D et 230E obtenues par imagerie sans lentille reflètent la taille des agglutinats, c'est-à-dire un degré d'agglutination. On comprend alors qu'en introduisant une quantité déterminée d'anticorps dans l'échantillon sanguin, il est possible d'estimer la quantité de protéine A présente dans cet échantillon en fonction de l'état d'agglutination, c'est-à-dire en fonction du deuxième indicateur $Ind2_A$, $Ind2_B$ précédemment décrit.

Autrement dit, la quantité de protéine A, au-delà de laquelle on observe une agglutination, constitue la limite de détection d'un dosage de cette protéine dans échantillon sanguin, en introduisant une quantité donnée d'anticorps dans l'échantillon de liquide à caractériser 12.

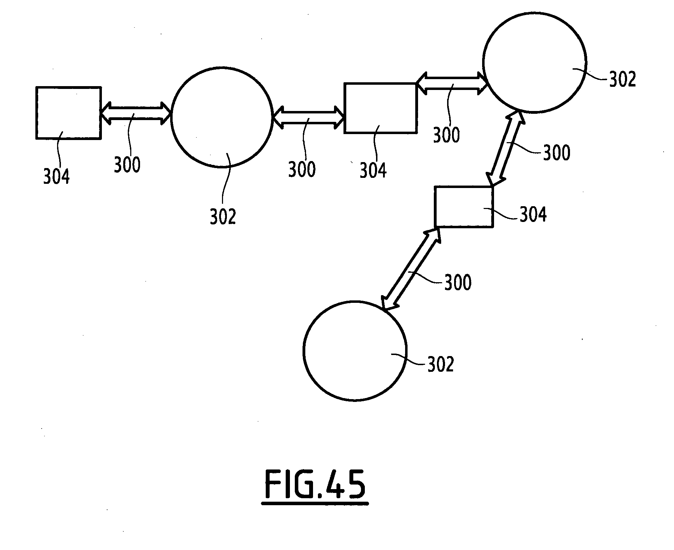

Ainsi, on comprend qu'il est possible d'observer, voire de quantifier, un état d'agglutination de particules dans un fluide biologique, par des indicateurs relatifs à l'image obtenue par imagerie sans lentille, et notamment le deuxième indicateur $Ind2_A$, $Ind2_B$, selon les première et deuxième variantes de ce deuxième exemple, qui est fonction de la distribution de l'intensité des pixels. Cet état d'agglutination dépend, par exemple, de la concentration d'un analyte dans le liquide biologique, la quantification de cet état d'agglutination permettant alors le dosage de cet analyte dans le liquide. L'exemple montre que ce dosage est réalisable en introduisant un réactif bi-fonctionnel, en l'occurrence l'anticorps 300, dans un échantillon sanguin, apte à se fixer à la fois sur une particule du fluide biologique, en l'occurrence les globules rouges 302, et sur l'analyte à doser, en l'occurrence la protéine A 304, formant ainsi un pontage entre un analyte 304 et les globules rouges 302, comme représenté sur la figure 45.

Le terme bi-fonctionnel désigne la faculté du réactif à se lier à la fois sur une particule et sur un analyte.

D'une façon générale, par analyte, on entend une espèce chimique ou biologique présente dans le liquide, telle qu'une molécule, une macromolécule (par exemple protéine ou acide nucléique), une cellule, une bactérie, un virus, ou encore un spore.

En outre, l'analyte 304 doit comporter au minimum 2 sites de liaison avec le réactif bi-fonctionnel, comme représenté sur la figure 45. Ainsi, chaque analyte 304 peut être lié, via le réactif bi-fonctionnel, à au moins deux particules. Cela entraîne l'agglutination des particules.

Autrement dit, l'état d'agglutination des particules dans le liquide 12 dépend de la quantité d'analyte présent dans le liquide 12, cette quantité étant dosable en ajoutant un réactif susceptible d'entraîner la formation d'agglutinats, le réactif 300 étant alors apte à se fixer entre une desdites particules 302 et un analyte 304 de façon à former un agglutinat.

En fonction de la quantité d'analyte 304 présent dans le liquide, on forme un agglutinat, composé de particules 302 et d'analytes 304. En déterminant l'état d'agglutination correspondant à une quantité donnée de réactif introduit, il est alors possible d'estimer la quantité d'analyte 304 présente dans le liquide 12.

Dans les deuxièmes et troisièmes exemples du deuxième mode de réalisation, décrits précédemment, la deuxième distance D2 est inférieure à 1 cm. Les inventeurs ont toutefois également observé que, dans le cas de la caractérisation de l'agglutination, des valeurs de la deuxième distance D2 supérieures à 1 cm, tels que des valeurs de quelques centimètres, voire quelques dizaines de centimètres, permettent d'obtenir des résultats exploitables, même si des valeurs de la deuxième distance D2 inférieures à 1 cm restent préférables.

D'une façon générale, ces deuxième et troisième exemples démontrent un autre aspect de l'invention. Selon cet autre aspect, l'invention concerne un procédé de caractérisation de l'agglutination de particules, telles des particules biologiques, dans un liquide, par exemple un liquide biologique, et en particulier un liquide corporel, le procédé de caractérisation comportant les étapes suivantes :

- l'introduction du liquide dans une chambre fluidique,
- l'éclairement de la chambre fluidique par un faisceau lumineux, le faisceau lumineux provenant en particulier d'une source de lumière, telle qu'une diode Laser ou une diode électroluminescente,
- l'acquisition d'une image, ou d'une pluralité d'images, de la chambre fluidique par un photodétecteur matriciel, le photodétecteur étant de préférence placé à une distance de la chambre fluidique inférieure à 1 cm, la chambre fluidique étant disposée entre la source de lumière et le photodétecteur matriciel,
- le traitement de l'image, ou de la pluralité d'images, pour déterminer un indicateur caractérisant l'agglutination de particules dans le liquide biologique, et
- la caractérisation de l'agglutination de particules dans le liquide, en fonction de la valeur de l'indicateur.

Il est à noter que l'image est acquise par le photodétecteur, de préférence sans optique de grossissement entre la chambre fluidique et le photodétecteur matriciel. Cependant, des microlentilles de focalisation peuvent être prévues au niveau de chaque pixel du détecteur, comme précédemment évoqué

En complément et de manière facultative, l'indicateur est un indicateur représentatif de la distribution de l'intensité des pixels dans une image, ou d'une façon plus générale, tout autre indicateur traduisant la segmentation de l'image selon différentes zones, chaque zone comportant plusieurs dizaines à centaines de pixels d'intensité comparable, c'est-à-dire dont l'intensité est répartie dans une plage de niveaux de gris de l'ordre de la moitié, voire du tiers, voire du quart, voire moins du quart de la dynamique de l'image.

En complément et de manière facultative, le procédé de caractérisation comporte l'ajout d'un réactif, susceptible d'entraîner l'agglutination de particules dans le liquide.

Comme cela est illustré dans le troisième exemple du deuxième mode de réalisation, l'agglutination des particules est, par exemple, fonction d'une quantité d'analyte présente dans le liquide.

Selon cette variante, l'invention concerne un procédé de détection de la quantité d'un analyte, dans un liquide, par exemple un liquide biologique, et en particulier un liquide corporel, le procédé de détection comportant les étapes suivantes :

- l'introduction du liquide dans une chambre fluidique,
- l'éclairement de la chambre fluidique par un faisceau lumineux, le faisceau lumineux provenant en particulier d'une source de lumière, telle qu'une diode Laser ou une diode électroluminescente,
- l'ajout d'un réactif, apte à entraîner la formation d'agglutinats de particules et d'analyte dans le liquide,
- l'acquisition d'une image, ou d'une pluralité d'images, de la chambre fluidique par un photodétecteur matriciel, le photodétecteur étant de préférence placé à une distance de la chambre fluidique inférieure à 1 cm, la chambre fluidique étant disposée entre la source de lumière et le photodétecteur matriciel,
- le traitement de l'image, ou de la pluralité d'images, pour déterminer un indicateur caractérisant l'agglutination de particules dans le liquide biologique, et
- l'estimation de la quantité d'analyte dans le liquide, en fonction de la valeur de l'indicateur.

Selon encore un autre aspect, l'invention concerne un procédé de détermination d'un paramètre du liquide 12, comportant du sang, le procédé comportant les étapes suivantes :

- l'introduction du liquide 12 dans la chambre fluidique 14,
- l'éclairage de la chambre fluidique 14 via le faisceau lumineux d'excitation 18 émis par la source de lumière 16, le faisceau lumineux 18 s'étendant à travers la chambre fluidique 14 selon la direction longitudinale X,
- l'acquisition d'au moins une image $I_n(x,y)$, $I_{n+m}(x,y)$, $I(x,y)$ par le photodétecteur matriciel 20, l'image $I_n(x,y)$, $I_{n+m}(x,y)$, $I(x,y)$ étant formée par un rayonnement transmis par la chambre fluidique 14 éclairée, et
- la détermination d'un indicateur $Ind1_{n,n+m}$, $Ind2$, à partir de ladite au moins une image $I_n(x,y)$, $I_{n+m}(x,y)$, $I(x,y)$.

Lors de l'étape d'acquisition, le photodétecteur 20 est disposé à la distance D2, inférieure à 1 cm, de la chambre fluidique 14 selon la direction longitudinale X.

En complément et de manière facultative, le procédé de détermination comprend une ou plusieurs des caractéristiques suivantes, prises isolément ou suivant toutes les combinaisons techniquement possibles :

- le faisceau lumineux 18 éclaire directement la chambre fluidique 14, et l'image $I_n(x,y)$, $I_{n+m}(x,y)$, $I(x,y)$ est formée directement par le rayonnement transmis par la chambre fluidique 14 éclairée, en l'absence d'une optique de grossissement disposée entre la chambre fluidique 14 et le photodétecteur 20 ;
- le paramètre est une coagulation, et le procédé comporte alors les étapes suivantes :

  + le mélange du liquide 12 avec un réactif pour favoriser la coagulation du sang,
  + l'acquisition d'une série d'image de transmission $I_n(x,y)$, $I_{n+m}(x,y)$ à des instants temporels n, n+m différents,
  + le calcul d'un indicateur $Ind1_{n,n+m}$ pour établir une corrélation entre deux zones des images de transmission $I_n(x,y)$, $I_{n+m}(x,y)$,
  la coagulation étant déterminée en fonction de la valeur dudit indicateur ;

- le paramètre est un temps de coagulation, et le procédé comporte alors les étapes suivantes :

  + le mélange du liquide 12 avec un réactif pour favoriser la coagulation du sang
  + l'acquisition d'une série d'image de transmission $I_n(x,y)$, $I_{n+m}(x,y)$ à des instants temporels n, n+m différents,
  + le calcul d'un indicateur $Ind1_{n,n+m}$ pour établir une corrélation entre deux images $I_n(x,y)$, $I_{n+m}(x,y)$, et
  + la détermination d'un intervalle temporel, dit temps de coagulation, entre un instant origine t0 et l'instant $t2_A$, $t2_B$ auquel l'indicateur $Ind1_{n,n+m}$ prend une valeur déterminée.

- le paramètre est une agglutination de particules sanguines, et le procédé comporte alors les étapes suivantes

  + le mélange du liquide 12 avec un réactif apte à engendrer une agglutination des particules sanguines,
  + l'acquisition d'une image de transmission $I(x,y)$,
  + le calcul d'un indicateur Ind2 en fonction de l'intensité dans une zone prédéterminée de l'image de transmission $I(x,y)$, et
  + la détermination d'un état d'agglutination lorsque cet indicateur Ind2 dépasse un seuil prédéterminé ;

- les particules sanguines sont des globules rouges, le réactif comportant un anticorps, l'état d'agglutination donnant alors une information relative au groupe sanguin.

Selon cet autre aspect indépendant, l'invention concerne également un système de détermination d'un paramètre du liquide 12, comportant du sang, le système de détermination comprenant :

- la chambre fluidique 14 destinée à recevoir le liquide 12;
- la source de lumière 16 propre à émettre le faisceau lumineux d'excitation 18 pour éclairer la chambre fluidique 14, le faisceau lumineux 18 s'étendant selon la direction longitudinale X;
- le photodétecteur matriciel 20 propre à acquérir au moins une image $I_n(x,y)$, $I_{n+1}(x,y)$, $I(x,y)$ d'un rayonnement transmis par la chambre fluidique 14 éclairée ; et
- l'unité de traitement d'informations 21 comportant des moyens de détermination d'un indicateur $Ind1_{n,n+m}$, Ind2, à partir de ladite au moins une image $I_n(x,y)$, $I_{n+m}(x,y)$, $I(x,y)$.

Le photodétecteur 20 est disposé à la distance D2, inférieure à 1 cm, de la chambre fluidique 14 selon la direction longitudinale X.

Le paramètre est une coagulation, un temps de coagulation, ou encore une agglutination de particules sanguines.

## Revendications

1.  Procédé de caractérisation d'une variation de la vitesse de particules ou d'une agglomération de particules, les particules, telles que des particules sanguines, étant contenues dans un liquide (12),
le procédé comportant les étapes suivantes :

    - l'introduction (100) du liquide (12) dans une chambre fluidique (14) ;
    - l'éclairage (120) de la chambre fluidique (14) via un faisceau lumineux d'excitation (18) émis par une source de lumière (16), le faisceau lumineux (18) s'étendant à travers la chambre fluidique (14) selon une direction longitudinale (X) ;
    - l'acquisition (130) d'au moins deux images ($I_n(x,y)$, $I_{n+m}(x,y)$, $I(x,y)$) par un photodétecteur matriciel (20), les

images ($I_n(x,y)$, $I_{n+m}(x,y)$, $I(x,y)$) étant formées par un rayonnement transmis par la chambre fluidique (14) éclairée ; et
- le calcul (140), à partir des images acquises ($I_n(x,y)$, $I_{n+m}(x,y)$, $I(x,y)$), d'au moins un indicateur ($Ind1_{n,n+m}$, $Ind2$) caractérisant la variation de la vitesse ou l'agglomération des particules ;

**caractérisé en ce que**, lors de l'étape d'acquisition (130), le photodétecteur (20) est disposé à une distance (D2) inférieure à 1 cm de la chambre fluidique (14) selon la direction longitudinale (X).

2. Procédé selon la revendication 1, dans lequel le faisceau lumineux (18) présente une surface comprise entre 5 mm$^2$ et 200 mm$^2$, de préférence égale à 25 mm$^2$, suivant un plan (P) perpendiculaire à la direction longitudinale (X), ledit plan (P) étant agencé au contact de la chambre fluidique (14).

3. Procédé selon la revendication 1 ou 2, dans lequel le faisceau lumineux (18) éclaire directement la chambre fluidique (14), et l'image ($I_n(x,y)$, $I_{n+m}(x,y)$ ; $I(x,y)$) est formée directement par le rayonnement transmis par la chambre fluidique (14) éclairée, en l'absence d'une optique de grossissement disposée entre la chambre fluidique (14) et le photodétecteur (20).

4. Procédé selon l'une quelconque des revendications précédentes, dans lequel, lors de l'étape d'acquisition (130), plusieurs images de transmission ($I_n(x,y)$, $I_{n+m}(x,y)$) sont acquises séquentiellement à des instants temporels (n, n+m) différents, et
dans lequel, lors de l'étape de calcul (140), un premier indicateur calculé ($Ind1_{n,n+m}$) est un indicateur de corrélation apte à caractériser la variation de la vitesse des particules, l'indicateur de corrélation ($Ind1_{n,n+m}$) étant représentatif de la corrélation entre au moins deux images de transmission ($I_n(x,y)$, $I_{n+m}(x,y)$), respectivement acquises aux instants n et n+m, ou de la corrélation pour une région prédéterminée desdites images de transmission ($I_n(x,y)$, $I_{n+m}(x,y)$).

5. Procédé selon la revendication 4, dans lequel le procédé comporte en outre une étape (110) de mélange du liquide (12) avec un réactif (112) apte à favoriser le ralentissement des particules, tel qu'un réactif (112) apte à favoriser le ralentissement des particules sanguines via une coagulation du sang.

6. Procédé selon la revendication 4 ou 5, dans lequel le procédé comporte en outre une étape de détermination, à partir du premier indicateur calculé ($Ind1_{n,n+m}$), de la coagulation des particules sanguines et/ou d'un intervalle temporel (Tc), dit temps de coagulation, entre un instant origine (t0) et l'instant ($t2_A$, $t2_B$) auquel le premier indicateur calculé ($Ind1_{n,n+m}$) prend une valeur prédéterminée.

7. Procédé selon l'une quelconque des revendications 4 à 6, dans lequel la source de lumière (16) est une source de lumière spatialement et temporellement cohérente, telle qu'un laser.

8. Procédé selon l'une quelconque des revendications 4 à 7, dans lequel le premier indicateur ($Ind1_{n,n+m}$) est calculé à partir d'une image de corrélation ($Icorr_{n,n+m}(x,y)$) définissant la corrélation spatiale entre lesdites images de transmission ($I_n$, $I_{n+m}$), ou d'une région prédéterminée (142) de ladite image de corrélation ($Icorr_{n,n+m}(x,y)$).

9. Procédé selon la revendication 8, dans lequel l'image de corrélation ($Icorr_{n,n+m}(x,y)$) est déterminée par l'équation suivante :

$$Icorr_{n,n+m}(x,y) = \frac{((A_n \times A_{n+m}) \otimes k1)(x,y)}{\sqrt{(A_n^2 \otimes k1)(x,y)}\sqrt{(A_{n+m}^2 \otimes k1)(x,y)}}$$

où x et y représentent les coordonnées d'un point de l'image, $Icorr_{n+m}(x,y)$ est une matrice ayant X lignes et Y colonnes,
k1 (x,y) représente une matrice prédéterminée ayant P lignes et Q colonnes,
$A_n(x,y)$ et $A_{n+m}(x,y)$ sont définis par les équations suivantes :

$$A_n(x,y) = I_n(x,y) - (I_n \otimes k1)(x,y)$$

$$A_{n+m}(x,y) = I_{n+m}(x,y) - (I_{n+m} \otimes k1)(x,y)$$

$I_n(x,y)$, $I_{n+m}(x,y)$ représentent deux images de transmission successives aux instants n et n+m, $I_n(x,y)$, $I_{n+m}(x,y)$ étant des matrices à X lignes et Y colonnes,
et le symbole $\otimes$ représente le produit de convolution défini par :

$$(F \otimes k1)(x,y) = \sum_{p=0}^{P}\sum_{q=0}^{Q} F(x-p, y-q)k1(p,q)$$

F(x,y) étant une matrice à X lignes et Y colonnes,
X, Y, P et Q étant des nombres entiers vérifiant $X \geq P \geq 1$ et $Y \geq Q \geq 1$.

**10.** Procédé selon l'une quelconque des revendications 4 à 7, dans lequel le premier indicateur ($Ind1_{n,n+m}$) est calculé à l'aide de l'équation suivante :

$$Ind1_{n,n+m} = \frac{\displaystyle\sum_{x=1,y=1}^{N,M} \sqrt{C_n^2(x,y)}\sqrt{C_{n+m}^2(x,y)}}{\sqrt{\displaystyle\sum_{x=1,y=1}^{N,M} C_n^2(x,y)}\sqrt{\displaystyle\sum_{x=1,y=1}^{N,M} C_{n+m}^2(x,y)}}$$

où $Ind1_{n,n+m}$ représente le premier indicateur,
$C_n(x,y)$ et $C_{n+m}(x,y)$ sont définis par les équations suivantes :

$$C_n(x,y) = I'_n(x,y) - \overline{I'_n}$$

$$C_{n+m}(x,y) = I'_{n+m}(x,y) - \overline{I'_{n+m}}$$

$I'_n(x,y)$, $I'_{n+m}(x,y)$ représentant respectivement une région prédéterminée de deux images de transmission successives aux instants n et n+m, x et y désignant les coordonnées d'un point de l'image, $I'_n(x,y)$, $I'_{n+m}(x,y)$ étant des matrices ayant N lignes et M colonnes, et
$\overline{I'}$ , $\overline{I'}$ représentant une valeur moyenne des régions prédéterminées respectives $I'_n(x,y)$ et $I'_{n+m}(x,y)$.

**11.** Procédé selon l'une quelconque des revendications 1 à 3, dans lequel le procédé comporte en outre une étape (110) de mélange du liquide (12) avec un réactif (206, 208) apte à engendrer une agglomération des particules, dans lequel, lors de l'étape de calcul (110), l'indicateur calculé est un deuxième indicateur (Ind2) pour chaque image acquise (I(x,y)), le deuxième indicateur (Ind2) étant représentatif de l'intensité des pixels de l'image (I(x,y)) dans une région prédéterminée de l'image (I(x,y)), et
dans lequel le procédé comporte en outre une étape de détermination d'un état d'agglomération des particules à partir du deuxième indicateur calculé (Ind2).

**12.** Procédé selon la revendication 11, dans lequel l'état d'agglomération est déterminé lorsque le deuxième indicateur (Ind2) dépasse un seuil prédéterminé.

**13.** Procédé selon la revendication 11, dans lequel l'état d'agglomération est déterminé lorsque le deuxième indicateur (Ind2) dépasse un indicateur de référence ($Ind2_{ref}$), obtenu par une image réalisée dans une zone de référence.

**14.** Procédé selon l'une quelconque des revendications 11 à 13, dans lequel les particules sanguines sont des globules rouges, le réactif (206, 208) comporte un anticorps, et une information relative au groupe sanguin est en outre déterminée à partir de l'état d'agglomération.

**15.** Procédé selon l'une quelconque des revendications 11 à 14, dans lequel le liquide (12) comporte un analyte, le procédé comportant alors l'estimation de la quantité dudit analyte dans le liquide (12), en fonction dudit deuxième indicateur (Ind2).

**16.** Procédé selon l'une quelconque des revendications précédentes, dans lequel la chambre fluidique (14) comporte plusieurs canaux (202, 204) de circulation du fluide, et dans lequel, lors de l'étape de calcul (140), un indicateur (Ind1$_{n,n+m}$, Ind2) est calculé pour chacun des canaux (202, 204).

**17.** Système (10) de caractérisation d'une variation de la vitesse de particules ou d'une agglomération de particules, les particules, telles que des particules sanguines, étant contenues dans un liquide (12), le système (10) comprenant :

- une chambre fluidique (14) destinée à recevoir le liquide (12) ;
- une source de lumière (16) propre à émettre un faisceau lumineux d'excitation (18) pour éclairer la chambre fluidique (14), le faisceau lumineux (18) s'étendant à travers la chambre fluidique (14) selon une direction longitudinale (X) ;
- un photodétecteur matriciel (20) propre à acquérir au moins deux images (I$_n$(x,y), I$_{n+1}$(x,y) ; I(x,y)) d'un rayonnement transmis par la chambre fluidique (14) éclairée ; et
- une unité de traitement d'informations (21) comportant des moyens de calcul (36, 38) adaptés pour calculer, à partir des images acquises (I$_n$(x,y), I$_{n+1}$(x,y), I(x,y)), au moins un indicateur (Ind1$_{n,n+m}$, Ind2) caractérisant la variation de la vitesse ou l'agglomération des particules ;
**caractérisé en ce que** le photodétecteur (20) est disposé à une distance (D2) inférieure à 1 cm de la chambre fluidique selon la direction longitudinale (X).

**18.** Système (10) selon la revendication 17, dans lequel le photodétecteur matriciel (20) comporte une pluralité de pixels, chaque pixel présentant des dimensions chacune inférieures ou égales à 4 $\mu$m.

**Patentansprüche**

**1.** Verfahren zur Charakterisierung einer Änderung der Geschwindigkeit von Partikeln oder von einer Agglomeration von Partikeln, wobei die Partikel, wie Blutpartikel, in einer Flüssigkeit (12) enthalten sind, wobei das Verfahren die folgenden Schritte umfasst:

- Einleiten (100) einer Flüssigkeit (12) in eine Fluidkammer (14);
- Beleuchten (120) der Fluidkammer (14) mittels eines Anregungslichtbündels (18), das von einer Lichtquelle (16) abgegeben wird, wobei das Lichtbündel (18) sich durch die Fluidkammer (14) gemäß einer Längsrichtung (X) erstreckt;
- Beschaffen (130) von mindestens zwei Bildern (I$_n$(x-y), I$_{n+m}$(x,y), I(x,y)) durch einen Matrixfotodetektor (20), wobei die Bilder (I$_n$(x-y), I$_{n+m}$(x,y), I(x,y)) durch eine Strahlung gebildet werden, die von der beleuchteten Fluidkammer (14) übertragen wird; und
- Berechnen (140) mindestens eines Indikators (Ind1$_{n,n+m}$. Ind2) aus den beschafften Bildern (I$_n$(x-y), I$_{n+m}$(x,y), I(x,y)), der die Änderung der Geschwindigkeit oder der Agglomeration der Partikel charakterisiert;

**dadurch gekennzeichnet, dass** bei dem Schritt des Beschaffens (130) der Fotodetektor (20) gemäß der Längsrichtung (X) in einer Entfernung (D2) kleiner als 1 cm von der Fluidkammer (14) angeordnet ist.

**2.** Verfahren nach Anspruch 1, bei dem das Lichtbündel (18) eine Fläche, die zwischen 5 mm$^2$ und 200 mm$^2$ liegt, vorzugsweise gleich 25 mm$^2$ ist, gemäß einer Ebene (P) senkrecht zur Längsrichtung (X) aufweist, wobei die Ebene (P) im Kontakt mit der Fluidkammer (14) angeordnet ist.

**3.** Verfahren nach Anspruch 1 oder 2, bei dem das Lichtbündel (18) direkt die Fluidkammer (14) beleuchtet und das Bild (I$_n$(x-y), I$_{n+m}$(x,y), I(x,y)) direkt durch die von der beleuchteten Fluidkammer (14) übertragenen Strahlung gebildet wird, in Abwesenheit einer zwischen der Fluidkammer (14) und dem Fotodetektor (20) angeordneten Vergrößerungsoptik.

**4.** Verfahren nach einem beliebigen der vorhergehenden Ansprüche, bei dem bei dem Schritt des Beschaffens (130) mehrere Übertragungsbilder (I$_n$(x-y), I$_{n+m}$(x,y)) aufeinanderfolgend bei unterschiedlichen zeitlichen Momenten (n, n+m) beschafft werden, und

bei dem bei dem Berechnungsschritt (140) ein erster berechneter Indikator (Ind1$_{n,n+m}$) ein Korrelationsindikator ist, der geeignet ist, die Änderung der Geschwindigkeit der Partikel zu charakterisieren, wobei der Korrelationsindikator (Ind1$_{n,n+m}$) repräsentativ für die Korrelation zwischen mindestens zwei Übertragungsbildern (I$_n$(x-y), I$_{n+m}$(x,y)), die jeweils zu den Zeitpunkten n und n+m erlangt wurden, oder für die Korrelation für einen vorbestimmten Bereich der Übertragungsbilder (I$_n$(x-y), I$_{n+m}$(x,y)) ist.

5. Verfahren nach Anspruch 4, bei dem das Verfahre außerdem einen Schritt (110) des Mischens der Flüssigkeit (12) mit einem Reagenz (112), das geeignet ist, die Verlangsamung der Partikel zu begünstigen, wie ein Reagenz (112), das geeignet ist, die Verlangsamung der Blutpartikel über eine Koagulation des Bluts zu begünstigen, umfasst.

6. Verfahren nach Anspruch 4 oder 5, bei dem das Verfahren außerdem einen Schritt des Bestimmens der Koagulation der Blutpartikel und/oder eines Zeitintervalls (Tc), genannt Koagulationszeit, zwischen einem Ursprungszeitpunkt (t0) und dem Zeitpunkt (t2$_A$, t2$_B$), bei dem der ersten berechnete Indikator (Ind1$_{n,n+m}$) einen vorbestimmten Wert annimmt, aus dem ersten berechneten Indikator (Ind1$_{n,n+m}$), umfasst.

7. Verfahren nach einem beliebigen der Ansprüche 4 bis 6, bei dem die Lichtquelle (16) eine zeitlich und räumlich kohärente Lichtquelle, wie ein Laser ist.

8. Verfahren nach einem beliebigen der Ansprüche 4 bis 7, bei dem der ersten Indikator (Ind1$_{n,n+m}$) aus einem Korrelationsbild (Icorr$_{n,n+m}$(x,y)), das die räumliche Korrelation zwischen den Übertragungsbildern (I$_n$, I$_{n+m}$) definiert, oder aus einem vorbestimmten Bereich (142) des Korrelationsbildes (Icorr$_{n,n+m}$(x,y)) berechnet wird.

9. Verfahren nach Anspruch 8, bei dem das Korrelationsbild (Icorr$_{n,n+m}$(x,y)) durch die folgende Gleichung bestimmt ist:

$$Icorr_{n,n+m}(x, y) = \frac{((A_n \times A_{n+m}) \otimes k1)(x, y)}{\sqrt{(A_n^2)(\otimes k1)(x, y)}\sqrt{((A_{n+m}^2) \otimes k1)(x, y)}}$$

wobei x und y die Koordinaten eines Bildpunktes bestimmen, Icorr$_{n,n+m}$(x,y) eine Matrix mit X Zeilen und Y Spalten ist, k1 (x,y) eine vorbestimmte Matrix mit P Zeilen und Q Spalten darstellt, A$_n$(x,y) und A$_{n+m}$(x,y) durch die folgenden Gleichungen definiert sind:

$$A_n(x, y) = I_n(x, y) - (I_n \otimes k1)(x, y)$$

$$A_{n+m}(x, y) = I_{n+m}(x, y9 - (I_{n+m} \otimes kl)(x, y)$$

wobei I$_n$(x,y), I$_{n+m}$(x,y) zwei aufeinanderfolgende Übertragungsbilder zu den Zeitpunkten n und n+m darstellen, wobei I$_n$(x,y), I$_{n+m}$(x,y) Matrizen mit X Zeilen und Y Spalten sind, und das Symbol $\otimes$ das Faltungsprodukt darstellt, das definiert ist durch:

$$F \otimes k1)(x, y) = \sum_{p=0}^{P} \sum_{q=0}^{Q} x - p, y - q)k1(p, q)$$

wobei F(x,y) eine Matrix mit X Zeilen und Y Spalten ist, X, Y, P und Q ganze Zahlen sind, die X≥P≥1 und Y≥Q≥1 verifizieren.

10. Verfahren nach einem beliebigen der Ansprüche 4 bis 7, bei dem der erste Indikator (Ind1$_{n,n+m}$) berechnet wird mit Hilfe der folgenden Gleichung:

$$Ind1_{n,n+m} = \frac{\sum\limits_{x=1,y=1}^{N,M}\sqrt{C_n^2(x,y)}\sqrt{C_{n+m}^2(x,y)}}{\sqrt{\sum\limits_{x=1,y=1}^{N,M}C_n^2(x,y)}\sqrt{\sum\limits_{y=1,y=1}^{N,M}C_{n+m}^2(x,y)}}$$

wobei $Ind1_{n,n+m}$ den ersten Indikator repräsentiert,
$C_n(x,y)$ und $C_{n+m}(x,y)$ durch die folgenden Gleichungen definiert sind:

$$C_n(x,y) = I'_n(x,y) = \overline{I'_n}$$

$$C_{n+m}(x,y) = I'_{n+m}(x,y) - \overline{I'_{n+m}}$$

wobei $I'_n(x,y)$, $I'_{n+m}(x,y)$ jeweils einen vorbestimmten Bereich von zwei aufeinanderfolgenden Übertragungsbildern zu den Zeitpunkten n und n+m darstellen, x und y die Koordinaten eines Bildpunkts bezeichnen, $I'_n(x,y)$, $I'_{n+m}(x,y)$ Matrizen sind, die N Zeilen und M Spalten haben, und $\overline{I'_n}$, $\overline{I'}$ einen Mittelwert der jeweiligen vorbestimmten Bereiche $I'_n(x,y)$ und $I'_{n+m}(x,y)$ darstellen.

11. Verfahren nach einem beliebigen der Ansprüche 1 bis 3, bei dem das Verfahren außerdem einen Schritt (110) des Mischens der Flüssigkeit (12) mit einem Reagenz (206, 208), das geeignet ist, eine Agglomeration der Partikel zu erzeugen, umfasst,
bei dem bei dem Berechnungsschritt (110) der berechnete Indikator ein zweiter Indikator (Ind2) für jedes erlange Bild (I(x,y)) ist, wobei der zweite Indikator (Ind2) repräsentativ für die Intensität der Pixel des Bildes (I(x,y)) in einem vorbestimmten Bereich des Bildes (I(x,y)) ist, und
bei dem das Verfahren außerdem einen Schritt der Bestimmens eines Agglomerationszustands der Partikel aus dem zweiten berechneten Indikator (Ind2) aufweist.

12. Verfahren nach Anspruch 11, bei dem der Agglomerationszustand bestimmt wird, wenn der zweite Indikator (Ind2) eine vorbestimmte Schwelle überschreitet.

13. Verfahren nach Anspruch 11, bei dem der Agglomerationszustand bestimmt wird, wenn der zweite Indikator (Ind2) einen Referenzindikator ($In2_{ref}$) überschreitet, der durch ein in einer Referenzzone realisiertes Bild erhalten wird.

14. Verfahren nach einem beliebigen der Ansprüche 11 bis 13, bei dem die Blutpartikel rote Blutkörperchen sind, das Reagenz (206, 208) einen Antikörper aufweist und eine Information bezüglich der Blutgruppe außerdem aus dem Agglomerationszustand bestimmt wird.

15. Verfahren nach einem beliebigen der Ansprüche 11 bis 14, bei dem die Flüssigkeit (12) einen Analyten aufweist, wobei das Verfahren dann die Schätzung der Menge des Analyten in der Flüssigkeit (12) abhängig vom zweiten Indikator (Ind2) umfasst.

16. Verfahren nach einem beliebigen der vorhergehenden Ansprüche, bei dem die Fluidkammer (14) mehrere Kanäle (202, 204) für die Zirkulation der Flüssigkeit aufweist und bei dem bei dem Schritt des Berechnens (140) ein Indikator ($Ind1_{n,n+m}$, Ind2) für jeden der Kanäle (202, 204) berechnet wird.

17. System (10) zur Charakterisierung einer Änderung der Geschwindigkeit der Partikel oder einer Agglomeration von Partikeln, wobei die Partikel, wie Blutpartikel, in einer Flüssigkeit (12) enthalten sind, wobei das System (10) umfasst:

- eine Fluidkammer (14), die zur Aufnahme der Flüssigkeit (12) dient;
- eine Lichtquelle (16), die geeignet ist, ein Anregungslichtbündel (18) zum Beleuchten der Fluidkammer (14) abzugeben, wobei das Lichtbündel (18) sich durch die Fluidkammer (14) gemäß einer Längsrichtung (X) erstreckt;
- einen Matrixfotodetektor (20), der geeignet ist, mindestens zwei Bilder ($I_n(x,y)$, $I_{n+1}(x,y)$, I(x,y)) einer von der beleuchteten Fluidkammer (14) übertragenen Strahlung zu beschaffen; und
- eine Informationsverarbeitungseinheit (21), die Mittel (36, 38) zur Berechnung aufweist, die geeignet sind, von den beschafften Bildern ($I_n(x,y)$, $I_{n+1}(x,y)$, $I(x,y)$) mindestens einen Indikator ($Ind1_{n,n+m}$, $Ind2$) zu berechnen, der die Änderung der Geschwindigkeit der Agglomeration der Partikel charakterisiert;

**dadurch gekennzeichnet, dass** der Fotodetektor (20) in einer Entfernung (D2) kleiner als 1 cm von der Fluidkammer gemäß der Längsrichtung (X) angeordnet ist.

18. System (10) nach Anspruch 17, bei dem der Matrixfotodetektor (20) eine Vielzahl von Pixeln aufweist, wobei jedes Pixel Abmessungen aufweist, die jeweils kleiner oder gleich 4 $\mu$m sind.

**Claims**

1. A method for characterizing a variation in the speed of particles or agglomeration of particles, the particles, such as blood particles, being contained in a liquid (12),
the method including the following steps:

   - introducing (100) the liquid (12) into a fluid chamber (14);
   - lighting (120) the fluid chamber (14) using an excitation light beam (18) emitted by a light source (16), the light beam (18) extending through the fluid chamber (14) in a longitudinal direction (X);
   - acquiring (130) at least two images ($I_n(x,y)$, $I_{n+m}(x,y)$, $I(x,y)$) using a matrix photodetector (20), the images ($I_n(x,y)$, $I_{n+m}(x,y)$, $I(x,y)$) being formed by radiation transmitted by the lighted fluid chamber (14); and
   - calculating (140), from the acquired images ($I_n(x,y)$, $I_{n+m}(x,y)$, $I(x,y)$), at least one indicator ($Ind1_{n,n+m}$, $Ind2$) characterizing the variation of the speed or agglomeration of the particles;

   **characterized in that**, during the acquisition step (130), the photodetector (20) is positioned at a distance (D2) smaller than 1 cm from the fluid chamber (14) in the longitudinal direction (X).

2. The method according to claim 1, wherein the light beam (18) has a surface area comprised between 5 mm$^2$ and 200 mm$^2$, preferably equal to 25 mm$^2$, in a plane (P) perpendicular to the longitudinal direction (X), said plane (P) being arranged in contact with the fluid chamber (14).

3. The method according to claim 1 or 2, wherein the light beam (18) directly lights the fluid chamber (14), and the image ($I_n(x,y)$, $I_{n+m}(x,y)$, $I(x,y)$) is formed directly by the radiation transmitted by the lighted fluid chamber (14), in the absence of a magnifying lens positioned between the fluid chamber (14) and the photodetector (20).

4. The method according to any one of the preceding claims, wherein, during the acquisition step (130), several transmission images ($I_n(x,y)$, $I_{n+m}(x,y)$) are acquired sequentially at different time instants (n, n+m), and wherein, during the calculation step (140), a first calculated indicator ($Ind1_{n,n+m}$) is a correlation indicator able to characterize the variation of the speed of the particles, the correlation indicator ($Ind1_{n,n+m}$) being representative of the correlation between at least two transmission images ($I_n(x,y)$, $I_{n+m}(x,y)$), respectively acquired at instants n and n+m, or the correlation for a predetermined region of said transmission images ($I_n(x,y)$, $I_{n+m}(x,y)$).

5. The method according to claim 4, wherein the method also includes a step (110) for mixing the liquid (12) with a reagent (112) capable of favoring slowing of the particles, such as a reagent (112) capable of favoring slowing of the blood particles by means of coagulation of the blood.

6. The method according to claim 4 or 5, wherein the method also includes a step for determining, from the first calculated indicator ($Ind1_{n,n+m}$), the coagulation of blood particles and/or a time interval (Tc), called coagulation time, between an initial instant (t0) and the instant ($t2_A$, $t2_B$) at which the first calculated indicator ($Ind1_{n,n+m}$) takes a predetermined value.

7. The method according to any one of claims 4 to 6, wherein the light source (16) is a spatially and temporally coherent light source, such as a laser.

8. The method according to any one of claims 4 to 7, wherein the first indicator ($Ind1_{n,n+m}$) is calculated from a correlation image ($Icorr_{n,n+m}(x,y)$) defining the spatial correlation between said transmission images ($I_n$, $I_{n+m}$), or from a prede-

termined region (142) of said correlation image ($Icorr_{n,n+m}(x,y)$).

9. The method according to claim 8, wherein the correlation image ($Icorr_{n,n+m}(x,y)$) is determined by the following equation:

$$Icorr_{n,n+m}(x,y) = \frac{\left(\left(A_n \times A_{n+m}\right) \otimes k1\right)(x,y)}{\sqrt{\left(\left(A_n^2\right) \otimes k1\right)(x,y)}\sqrt{\left(\left(A_{n+m}^2\right) \otimes k1\right)(x,y)}}$$

where x and y represent the coordinates of a point of the image, $Icorr_{n,n+m}(x,y)$ is a matrix having X rows and Y columns,
$k1(x,y)$ represents a predetermined matrix having P rows and Q columns,
$A_n(x,y)$ and $A_{n+m}(x,y)$ are defined by the following equations:

$$A_n(x,y) = I_n(x,y) - \left(I_n \otimes k1\right)(x,y)$$

$$A_{n+m}(x,y) = I_{n+m}(x,y) - \left(I_{n+m} \otimes k1\right)(x,y)$$

$I_n(x,y)$, $I_{n+m}(x,y)$ representing two successive transmission images at instants n and n+m, $I_n(x,y)$, $I_{n+m}(x,y)$ being matrices with X rows and Y columns,
and the symbol $\otimes$ represents the convolution defined by:

$$\left(F \otimes k1\right)(x,y) = \sum_{p=0}^{P}\sum_{q=0}^{Q} F(x-p,y-q)k1(p,q)$$

$F(x,y)$ being a matrix with X rows and Y columns,
X, Y, P and Q being integers verifying $X \geq P \geq 1$ and $Y \geq Q \geq 1$.

10. The method according to any one of claims 4 to 7, wherein the first indicator ($Ind1_{n,n+m}$) is calculated using the following equation:

$$Ind1_{n,n+m} = \frac{\sum_{x=1,y=1}^{N,M}\sqrt{C_n^2(x,y)}\sqrt{C_{n+m}^2(x,y)}}{\sqrt{\sum_{x=1,y=1}^{N,M}C_n^2(x,y)}\sqrt{\sum_{x=1,y=1}^{N,M}C_{n+m}^2(x,y)}}$$

where $Ind1_{n,n+m}$ represents the first indicator,
$C_n(x,y)$ and $C_{n+m}(x,y)$ are defined by the following equations:

$$C_n(x,y) = I'_n(x,y) - \overline{I'_n}$$

$$C_{n+m}(x,y) = I'_{n+m}(x,y) - \overline{I'_{n+m}}$$

$I'_n(x,y)$, $I'_{n+m}(x,y)$ respectively represent a predetermined region of two successive transmission images at instants n and n+m, x and y designating the coordinates of a point of the image, $I'_n(x,y)$, $I'_{n+m}(x,y)$ being matrices having N rows and M columns, and
$\overline{I'}$, $\overline{I'}$ representing a mean value of respective predetermined regions $I'_n(x,y)$ and $I'_{n+m}(x,y)$.

11. The method according to any one of claims 1 to 3, wherein the method also includes a step (110) for mixing the liquid (12) with a reagent (206, 208) capable of creating an agglomeration of particles,
wherein, during the calculation step (110), the calculated indicator is a second indicator (Ind2) for each acquired image (I(x,y)), the second indicator (Ind2) being representative of the intensity of the pixels of the image (I(x,y)) in a predetermined region of the image (I(x,y)), and
wherein the method also includes a step for determining an agglomeration state of the particles from the second calculated indicator (Ind2).

12. The method according to claim 11, wherein the agglomeration state is determined when the second indicator (Ind2) exceeds a predetermined threshold.

13. The method according to claim 11, wherein the agglomeration state is determined when the second indicator (Ind2) exceeds a reference indicator (Ind2$_{ref}$), obtained by an image made in a reference area.

14. The method according to any one of claims 11 to 13, wherein the blood particles are red blood cells, the reagent (206, 208) includes an antibody, and a piece of information relative to the blood group is also determined from the agglomeration state.

15. The method according to any one of claims 11 to 14, wherein the liquid (12) includes an analyte, the method then including estimating the quantity of said analyte in the liquid (12), as a function of said second indicator (Ind2).

16. The method according to any one of the preceding claims, wherein the fluid chamber (14) includes several fluid circulation channels (202, 204), and wherein, during the calculation step (140), an indicator (Ind1$_{n,n+m}$, Ind2) is calculated for each of the channels (202, 204).

17. A system (10) for characterizing the variation of the speed of particles or the agglomeration of particles, the particles, such as blood particles, being contained in the liquid (12), the system (10) comprising:

  - a fluid chamber (14) designed to receive the liquid (12);
  - a light source (16) capable of emitting an excitation light beam (18) to light the fluid chamber (14), the light beam (18) extending through the fluid chamber (14) in a longitudinal direction (X);
  - a matrix photodetector (20) capable of acquiring at least two images (I$_n$(x,y), I$_{n+1}$(x,y), I(x,y)), of a radiation transmitted by the lighted fluid chamber (14); and
  - an information processing unit (21) including calculation means (36, 38) for calculating, from the acquired images (I$_n$(x,y), I$_{n+1}$(x,y), I(x,y)), at least one indicator (Ind1$_{n,n+m}$, Ind2) characterizing the variation of the speed or the agglomeration of the particles;

  **characterized in that** the photodetector (20) is positioned at a distance (D2) smaller than 1 cm from the fluid chamber in the longitudinal direction (X).

18. The system (10) according to claim 17, wherein the matrix photodetector (20) includes a plurality of pixels, each pixel having dimensions each smaller than or equal to 4 $\mu$m.

FIG.1

EP 2 669 678 B1

**FIG.2**

## FIG.3

## FIG.4

| Introduction du liquide dans la chambre fluidique | ⟋100 |

| Mélange éventuel du liquide avec un réactif | ⟋110 |

| Eclairage de la chambre fluidique par un faisceau lumineux | ⟋120 |

| Acquisition, par un photodétecteur matriciel, d'image(s) de la chambre fluidique éclairée | ⟋130 |

| Calcul d'un indicateur caractérisant le ralentissement ou l'agglomération de particules du liquide, à partir de la ou des images acquises | ⟋140 |

## FIG.5

FIG.6

FIG.7

FIG.8

FIG.9

FIG.10

FIG.11

EP 2 669 678 B1

Groupe sanguin du patient

| | | A | B | AB | O |
|---|---|---|---|---|---|
| **Anticorps déposé** | Anti-A (sérum B) | ✕ | | ✕ | |
| | Anti-B (sérum A) | | ✕ | ✕ | |

✕ = agrégation cellulaire

## FIG.12

## FIG.13

**FIG.14**

**FIG.15**

## FIG.16

## FIG.17

220A

**FIG.18**

220B

**FIG.19**

220C

**FIG.20**

220D

**FIG.21**

222A

**FIG.22**

222B

**FIG.23**

222C

**FIG.24**

222D

**FIG.25**

224A

## FIG.26

224B

## FIG.27

224C

## FIG.28

224D

## FIG.29

230A

FIG.30

230B

FIG.31

230C

FIG.32

230D

FIG.33

230E

FIG.34

232A

FIG.35

232B

FIG.36

232C

FIG.37

232D

FIG.38

232E

FIG.39

234A

FIG.40

234B

FIG.41

234C

FIG.42

234D

FIG.43

234E

FIG.44

**FIG.45**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2233923 A1

**Littérature non-brevet citée dans la description**

- **KALCHENKO, V. et al.** *Journal of Biomedical Optics,* 2007, vol. 12 (5), 052002

- **PIEDERRIÈRE, Y et al.** *Journal of Biomedical Optics,* 2004, vol. 9 (2), 408-412